# EUROPEAN PATENT SPECIFICATION

(11) **EP 3 313 402 B1**
(45) Date of publication and mention of the grant of the patent: **27.12.2023**
(21) Application number: 16734150.2
(22) Date of filing: 23.06.2016
(51) Int. Cl.: A61K 31/437, A61P 25/18

(54) **VMAT2 INHIBITORS FOR TREATING NEUROLOGICAL DISEASES OR DISORDERS**
VMAT2-INHIBITOREN ZUR BEHANDLUNG VON NEUROLOGISCHEN KRANKHEITEN ODER STÖRUNGEN
INHIBITEURS DE VMAT2 POUR LE TRAITEMENT DE MALADIES OU TROUBLES NEUROLOGIQUES

(30) Priority: 23.06.2015 US 201562183519 P; 23.06.2015 US 201562183520 P; 23.06.2015 US 201562183525 P; 23.06.2015 US 201562183530 P; 30.10.2015 US 201562248797 P; 30.10.2015 US 201562248803 P; 04.11.2015 US 201562251007 P; 04.11.2015 US 201562251009 P; 04.11.2015 US 201562251012 P; 04.11.2015 US 201562251018 P; 04.11.2015 US 201562251019 P; 04.11.2015 US 201562251023 P; 03.12.2015 US 201562262856 P; 03.12.2015 US 201562262860 P; 03.02.2016 US 201662290839 P; 03.02.2016 US 201662290864 P
(43) Date of publication of application: 02.05.2018
(73) Proprietor: NEUROCRINE BIOSCIENCES, INC., San Diego, CA 92130-1102 (US)
(72) Inventor: O'BRIEN, Christopher, F., San Diego, California 92130-1102 (US); GRIGORIADIS, Dimitri E., San Diego, California 92130-1102 (US)
(74) Representative: Mewburn Ellis LLP
(86) International application number: PCT/US2016/039098
(87) International publication number: WO 2016/210180

(56) References cited:
- WO-A1-2008/058261
- WO-A1-2014/047167
- WATTS ET AL: "Clinical and biochemical studioes on treatment of Lesch-Nylan syndrome", ARCHIVES OF DISEASE IN CHILDHOOD., vol. 49, 1974, pages 693-702, XP002764462, DOI: 10.1136/adc.49.9.693
- JANKOVIC ET AL: "Lesch-Nylan Syndrome. A Study of Motor Behaviour and Cerebrospinal Fluid Neurotransmitters", ANN. NEURO., vol. 23, no. 5, May 1988 (1988-05), pages 466-469, XP002764463, DOI: 10.1002/ana.410230507
- GUILLOTEAU D ET AL: CURRENT PHARMACEUTICAL DESIGN, vol. 11, no. 25, 1 January 2005 (2005-01-01), pages 3237-3245, XP009192465, BENTHAM SCIENCE PUBLISHERS, NL ISSN: 1381-6128
- Vivienne Shen ET AL: "Articles Safety and Efficacy of Tetrabenazine and Use of Concomitant Medications During Long-Term, Open-Label Treatment of Chorea Associated with Huntington's and Other Diseases", , 22 October 2013 (2013-10-22), XP055321497, Retrieved from the Internet: URL:https://www.ncbi.nlm.nih.gov/pmc/artic les/PMC3822048/pdf/tre-03-191-4337-1.pdf [retrieved on 2016-11-22]

## Description

### BACKGROUND

### Technical Field

Provided herein is a VMAT2 inhibitor for use, in a subject in need thereof, in methods of treating certain neurological diseases or disorders relating in part to monoamine imbalance, including mania in mood disorders, depression in mood disorder and treatment-refractory obsessive compulsive disorder

### Description of the Related Art

Mood disorders represent a category of mental disorders in which the underlying problem primarily affects a person's persistent emotional state (their mood). Mood disorders include: major depressive disorder (also called major depression), bipolar disorder, persistent depressive disorder (long lasting low grade depression), cyclothymia (a mild form of bipolar disorder), catatonic depression, post-partum depression, mania, and seasonal affective disorder (SAD). Mood disorders include substance-induced mood disorders and mood disorders due to a medical condition, *e.g.*, hypothyroidism or Parkinson's disease.

Bipolar disorder, also known as bipolar affective disorder or manic-depressive illness, is a mental disorder characterized by periods of elevated mood and periods of depression. The periods of elevated mood is known as mania or hypomania depending on the severity or whether psychosis is present. Symptoms of mania or a manic episode include a long period of feeling "high" or an overly happy or outgoing mood, extreme irritability, talking very fast, racing thoughts, jumping from one idea to another, being easily distracted, increasing activities, being overly restless, sleeping little, having an unrealistic belief in one's abilities, impulsive behavior, and engaging in pleasurable, high-risk behaviors. Symptoms of depression or a depressive episode include: an overly long period of sadness or hopelessness, loss of interest in activities, feeling tired, problems with concentration or memory, difficulty making decisions, being restless or irritable, change in eating or sleeping habits, and suicide ideation. Patients with bipolar disorder have a high risk of suicide and self-harm. The cause of bipolar disorder is not completely understood, but both genetic and environmental factors are thought to play a role. Environmental factors include long term stress and a history of child abuse.

Medications for treatment of the manic, psychotic, or depressive aspects of bipolar disorder generally include mood stabilizers, atypical antipsychotics, or antidepressants, in combination with psychotherapy. Sleep medications may also be used to help with sleep disturbances. For severe cases in which medication and psychotherapy does not work, electroconvulsive therapy may be used. Bipolar disorder usually is a lifelong illness and can worsen if left untreated. Long-term, continuous treatment is needed to control symptoms, and even with proper treatment mood changes can still occur. Patients frequently need to try several different medications before finding ones that help control symptoms. Given the unpleasant and potentially severe side effects associated with these medications, particularly anti-psychotic medications, a need exists to develop new therapeutics for treating mania in mood disorders and their related symptoms.

Obsessive-compulsive disorder (OCD) is an anxiety disorder characterized by recurrent and persistent anxiety-provoking thoughts (obsessions) that lead to repetitive behaviors (compulsions) that focus on alleviating distress caused by obsessive thoughts. Patients may or may not recognize that the obsessions and compulsions are unreasonable, and these thoughts and behaviors can become time-consuming and impair function.

OCD is generally treated with psychotherapy, medication or both. Cognitive behavior therapy (CBT) teaches a person different ways of thinking, behaving, and reacting to situations that help him or her to feel less anxious or fearful without having obsessive thoughts or acting compulsively (cognitive restructuring and exposure response prevention). However, CBT takes effort and practice to learn healthy ways to cope with anxiety. Medications may also be prescribed to treat OCD. The most commonly prescribed medications are anti-anxiety medications and anti-depressants. Anti-anxiety medications begin working right away, but should not be taken for long periods of time. Anti-depressants may take 10 to 12 weeks to start working and can cause side effects such as headache, nausea, sleep disturbance, and reduced libido. Atypical anti-psychotics may also be prescribed. It is not unusual for OCD patients to have to try several medications before finding one that controls OCD symptoms.

However, even when OCD is appropriately diagnosed and treated, many OCD patients are "treatment-resistant" or "treatment-refractory" and do not adequately respond to standard therapies. An estimated 10% to 40% of OCD patients are treatment-refractory (Bystritsky, Mol. Psychiatry 11:805-814). Treatment resistance generally refers to a lack of sufficient improvement despite multiple adequate and appropriate treatment trials. For mood disorders, it may be defined by failure to remit or respond clinically (50% reduction in symptoms) despite ≥ 2 adequate antidepressant trials or failure to respond clinically despite adequate medication trials across several neurotransmitter classes. Pallanti and Quercioli (Neuropsychopharmacol. Biol. Psychiatry 30:400-412) proposed categorizing obsessive-compulsive disorder treatment response into several stages along a spectrum, ranging from complete recovery (or remission) to full or partial response to non-response (or completely refractory). Whichever definition is used, patients with treatment resistance in anxiety disorders experience minimal restoration of function despite several appropriate treatment exposures. Factors that contribute to treatment resistance in OCD include, but are not limited to, disease severity, medical comorbidity, psychiatric comorbidity, treatment non-compliance, cultural factors, childhood stressors, long-term persistent stressors, life stage, and limitations of clinician/health system (Khalsa et al., Curr. Psychiatry, 2011, 10:45-52). Invasive therapies, including some that are irreversible, such as electroconvulsive therapy, vagal nerve stimulation, repetitive transcranial magnetic stimulation, and surgical methods, are reserved for patients with the strongest treatment resistance. More effective treatments are therefore needed to treat the symptoms associated with treatment refractory OCD.

Depression is a common feature of mental illness, whatever its nature and origin. A person with a history of any serious psychiatric disorder has almost as high a chance of developing major depression as someone who has had major depression itself in the past. About 20% of the U.S. population reports at least one depressive symptom in a given month, and 12% report two or more in a year. Mood disorders represent a category of mental disorders in which the underlying problem primarily affects a person's persistent emotional state (their mood). Bipolar disorder is less common, occurring at a rate of 1% in the general population, but some believe the diagnosis is often overlooked because manic elation is too rarely reported as an illness. Bipolar disorder is an illness involving one or more episodes of serious mania and depression. Sometimes a person might only experience symptoms of mania. If a person only experiences feelings of sadness, this is considered depression. During episodes of bipolar disorder, a person's mood can swing from excessively "high" and/or irritable to sad and hopeless, with periods of a normal mood in between.

Major depressive disorder is one of the most common mental illnesses. Depression causes people to lose pleasure from daily life, can complicate other medical conditions, and can even be serious enough to lead to suicide. Depression can occur to anyone, at any age, and to people of any race or ethnic group. Depression is usually treated with medications, psychotherapy, or a combination of the two. Medications for major depressive disorder fall in multiple drug classes, including tricyclic antidepressants, monoamine oxidase inhibitors, selective serotonin reuptake inhibitors, and atypical antidepressants. However, most antidepressants require at least 4-6 weeks for onset of effectiveness, and many antidepressants have unpleasant side effects. Moreover, as many as two-thirds of patients with depression experience treatment failure with the first anti-depressant, and up to a third of patients with depression don't respond to several attempts at treatment. Given the unpleasant and potentially severe side effects associated with these medications, a need exists to develop new therapeutics for treating depression in mood disorders and their related symptoms.

Provided by the present disclosure are approaches and embodiments addressing such needs, and further providing other related advantages.

### BRIEF SUMMARY

The present invention is defined in the appended claims.

In particular embodiments, the uses described herein comprise administering a pharmaceutical composition comprising at least one pharmaceutically acceptable excipient and a VMAT2 inhibitor.

These and other aspects of the invention will be apparent upon reference to the following detailed description. To this end, various references are set forth herein that describe in more detail certain background information, procedures, compounds and/or compositions.

Terms not specifically defined herein should be given the meanings that would be given to them by one of skill in the art in light of the disclosure and the context. As used in the specification, however, unless specified to the contrary, the terms have the meaning indicated.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Also, as used in this specification and the appended claims, the singular forms "a," "an," and "the" include plural referents unless the content clearly dictates otherwise. Thus, for example, reference to "a non-human animal" may refer to one or more non-human animals, or a plurality of such animals, and reference to "a cell" or "the cell" includes reference to one or more cells and equivalents thereof (e.g., plurality of cells) known to those skilled in the art, and so forth. When steps of a method are described or claimed, and the steps are described as occurring in a particular order, the description of a first step occurring (or being performed) "prior to" *(i.e.,* before) a second step has the same meaning if rewritten to state that the second step occurs (or is performed) "subsequent" to the first step. The term "about" when referring to a number or a numerical range means that the number or numerical range referred to is an approximation within experimental variability (or within statistical experimental error), and thus the number or numerical range may vary between 1% and 15% of the stated number or numerical range. It should also be noted that the term "or" is generally employed in its sense including "and/or" unless the content clearly dictates otherwise. The term, "at least one," for example, when referring to at least one compound or to at least one composition, has the same meaning and understanding as the term, "one or more."

### BRIEF DESCRIPTION OF THE DRAWING

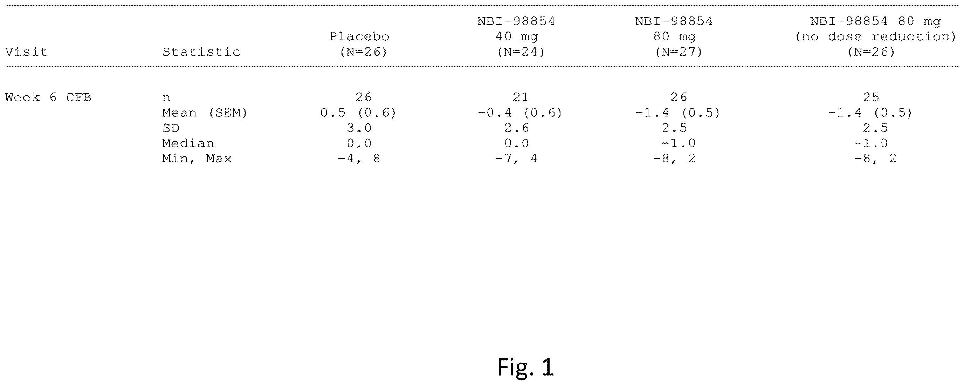

**Figure 1** shows Young Mania Rating Scale Total Score and Change from Baseline (CFB) Values in subjects with underlying mood disorders receiving placebo or (S)-2-amino-3-methyl-butyric acid (2R,3R, 11bR)-3-isobutyl-9,10-dimethoxy-1,3,4,6,7,11b-hexahydro-2H-pyrido[2,1-a]isoquinolin-2-yl ester ditosylate (valbenazine ditosylate, also known as NBI-98854) treated at week 6 of treatment.

### DETAILED DESCRIPTION

The present disclosure is directed to methods of treating diseases or disorders relating to a neurotransmitter imbalance, particularly monoamine imbalance. Monoamine neurotransmitters contain one amino group that is connected to an aromatic ring by a two-carbon chain. Monoamine neurotransmitters include, for example, catecholamines (*e.g.*, dopamine, norepinephrine), tryptamines *(e.g.,* serotonin), and histamine. VMAT2 is a transporter which is present in the membrane of presynaptic vesicles in monoaminergic neurons. Their primary function appears to be packaging monoamines (*e.g.*, dopamine) from the cytoplasm of the presynaptic neuron into vesicles for subsequent release into the synapse. Inhibition of VMAT2 results in modulated activity in associated neuronal circuits.

Without wishing to be bound by theory, monoamine imbalance, for example excessive dopamine activity, may contribute to certain behavioral symptoms, for example, repetitive, involuntary physical behaviors and stereotypies. One approach for modulating dopaminergic pathways in the brain is to use dopamine 2 receptor (D2) antagonist drugs. However, some patients fail to respond to D2 receptor antagonists, and they are often poorly tolerated due to their side effects (*e.g.*, sedation, depression, weight gain, metabolic syndrome). Another approach is to use tetrabenazine. Tetrabenazine is a VMAT2 inhibitor that depletes presynaptic dopamine. However, tetrabenazine is poorly tolerated due to the side effects of excessive dopamine depletion, including sedation, depression, akathisia, and parkinsonism. Moreover, tetrabenazine has the potential to cause tardive dyskinesia due to off-target binding by one of the stereoisomer metabolites to the post-synaptic D2 receptor. Other drawbacks to tetrabenazine treatment are the fluctuating response and the need for frequent intake due to TBZ rapid metabolism. Therefore, there is an unmet need in the art to develop effective therapeutics for treatment of neurological disorders, including mania or depression in mood disorders, treatment-refractory OCD, agitation in Alzheimer's disease, autism spectrum disorder, Lesch-Nyhan disease, Fragile X syndrome, Rett syndrome, and chorea acanthocytosis.

The present disclosure provides compositions and methods for monoamine stabilization for treating neurological diseases or disorders relating in part to a neurotransmitter imbalance, particularly monoamine imbalance, using a VMAT2 inhibitor.

In one aspect, unexpectedly a VMAT2 inhibitor may be useful in methods for treating mania in mood disorders, including bipolar disorder. In certain embodiments, the mania in bipolar disorder to be treated is hypomania or severe mania. Any one of the VMAT2 inhibitors described herein may treat one or more problem moods or behaviors associated with mania in mood disorders. By way of example, a VMAT2 inhibitor may be used to treat problem moods or behaviors associated mania in bipolar disorder, including overly excited or joyful state, extreme irritability, racing thoughts, flight of ideas, psychomotor agitation, talking very fast, increased talkativeness, increased restlessness, increase in goal-directed activity, decreased need for sleep, inflated self-esteem or grandiosity, impulsive behavior, and excessive involvement in pleasurable activities that have a high potential for painful consequence. VMAT2 inhibition results in modulation of the neurotransmitter systems (*e.g.*, dopamine and serotonin). Data from a variety of domains, *e.g.*, genetic, pharmacology, and neuroimaging, suggest a role for increased dopamine transmission in mania, and as such, a reduction in intensity, frequency, and amplitude of mania in bipolar disorder would be measurable on a variety of clinical assessment scales.

In another aspect, unexpectedly a VMAT2 inhibitor may be useful in methods for treating treatment-refractory obsessive-compulsive disorder (OCD). Any one of the VMAT2 inhibitors described herein may reduce one or more obsessive-compulsive behaviors, including a cleaning, hoarding, a counting ritual, a checking ritual, a line-crossing, a prayer ritual, a hand washing ritual, following a strict routine, orderliness, requesting reassurance, or a combination thereof. VMAT2 inhibition results in modulation of neurotransmitter systems (*e.g.*, dopamine and serotonin), which appear to be connected with the pathophysiology of OCD and, as such, a reduction in frequency and severity of the various OCD behavior intensity, frequency, and amplitude would be measurable on a variety of clinical assessment scales.

In another aspect, unexpectedly a VMAT2 inhibitor may be used for treating depression in mood disorders.

In certain embodiments, the mood disorder bipolar disorder or major depressive disorder. Any one of the VMAT2 inhibitors described herein may treat one or more problem moods or behaviors associated with depression in mood disorders. By way of example, a VMAT2 inhibitor may be used to treat problem moods or behaviors associated depression in a mood disorder, including persistent sadness, anxiety or "empty" mood, sleeping too much or too little, middle of the night or early morning waking, reduced appetite and weight loss or increased appetite and weight gain, loss of pleasure and interest in activities once enjoyed, including sex, restlessness, irritability, persistent physical symptoms that do not respond to treatment (such as chronic pain or digestive disorders), difficulty concentrating, remembering, or making decisions, fatigue or loss of energy, feeling guilty, hopeless or worthless, and suicide ideation. VMAT2 inhibition results in modulation of the neurotransmitter systems (e.g., dopamine and serotonin). Genetic and pharmacological data suggest a role for monoamines in the features of depression, and as such, a reduction in intensity, frequency, and amplitude of depression in a mood disorder would be measureable on a variety of clinical assessment scales.

### VMAT2 Inhibitors

The term "VMAT2" also known as "solute carrier family 18 member 2" (SLC18A2) refers to human vesicular monoamine transporter isoform 2, an integral membrane protein that acts to transport monoamines, particularly neurotransmitters such as dopamine, norepinephrine, serotonin, and histamine, from cellular cytosol into synaptic vesicles.

The terms "active ingredient" and "active substance" refer to a compound, which is administered, alone or in combination with one or more pharmaceutically acceptable excipients, to a subject for treating, preventing, or ameliorating one or more symptoms of a disorder, disease, or condition. In certain embodiments, "active ingredient" and "active substance" may be an optically active isomer or an isotopic variant of a compound described herein.

The terms "drug," "therapeutic agent," and "chemotherapeutic agent" refer to a compound, or a pharmaceutical composition thereof, which is administered to a subject for treating, preventing, or ameliorating one or more symptoms of a disorder, disease, or condition.

The term "VMAT2 inhibitor", "inhibit VMAT2", or "inhibition of VMAT2" refers to the ability of a compound disclosed herein to alter the function of VMAT2. A VMAT2 inhibitor may block or reduce the activity of VMAT2 by forming a reversible or irreversible covalent bond between the inhibitor and VMAT2 or through formation of a noncovalently bound complex. Such inhibition may be manifest only in particular cell types or may be contingent on a particular biological event. The term "VMAT2 inhibitor", "inhibit VMAT2", or "inhibition of VMAT2" also refers to altering the function of VMAT2 by decreasing the probability that a complex forms between a VMAT2 and a natural substrate. In some embodiments, modulation of the VMAT2 may be assessed using the method described in WO 2005077946; WO 2008/058261; EP 1716145; Kilbourn et al., European Journal of Pharmacology 1995, (278), 249-252; Lee et al., J. Med. Chem., 1996, (39), 191-196; Scherman et al., Journal of Neurochemistry 1988, 50(4), 1131-36; Kilbourn et al., Synapse 2002, 43(3), 188-194; Kilbourn et al., European Journal of Pharmacology 1997, 331(2-3), 161-68; and Erickson et al., Journal of Molecular Neuroscience 1995, 6(4), 277-87. In certain embodiments, a VMAT2 inhibitor does not include tetrabenazine. In certain embodiments, a VMAT2 inhibitor is a selective VMAT2 inhibitor that does not exhibit significant inhibition of human vesicular monoamine transporter type 1 (VMAT1), dopamine receptor type 2 (D2), or serotonin receptor. In certain embodiments, selective VMAT2 inhibitors are less likely to exhibit "off-target" side effects or excessive monoamine depletion, which can cause sedation, depression akathisia, and parkinsonism. As used herein, tetrabenazine is not a selective VMAT2 inhibitor. In certain embodiments, a selective VMAT2 inhibitor is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester or a salt thereof (e.g., ditosylate salt), or [(2R,3S,11bR)-9,10-Dimethoxy-3-(2-methylpropyl)-1H,2H,3H,4H,6H,7H,11bH-pyrido[2,1-a]isoquinolin-2-yl]methanol or a prodrug thereof.

VMAT2 inhibitors (and isotopic variants, polymorphs, physiologically acceptable salts thereof) may reduce the supply of monoamines in the central nervous system by inhibiting vesicular monoamine transporter isoform 2 (VMAT2). Examples of VMAT2 inhibitors and monoamine depletors that may be used in the methods described herein for treating mania in mood disorders, treatment refractory OCD and depression in mood disorders, include, for example, tetrabenazine (3-isobutyl-9,10-dimethoxy-1,3,4,6,7,11b-hexahydro-2H-pyrido[2,1-a]isoquinolin-2-one, TBZ). TBZ is approved for the treatment of chorea associated with Huntington's disease. Use of tetrabenazine for the treatment of tardive dyskinesia and a variety of hyperkinetic movement disorders has also been described. Tetrabenazine is readily metabolized upon administration to dihydrotetrabenazine (3-isobutyl-9,10-dimethoxy-1,3,4,6,7, 11b-hexahydro-2H-pyrido[2,1-a]isoquinolin-2-ol, DHTBZ), with the R,R,R stereoisomer of DHTBZ believed to be the most active metabolite. In certain embodiments, a VMAT2 inhibitor for use in the methods described herein does not include tetrabenazine. In this invention, a VMAT 2 inhibitor for use in the methods described herein is a selective VMAT2 inhibitor.

In certain embodiments, the methods described herein for treating mania in mood disorders comprise administering TBZ analogs and metabolites, lobeline and analogs, and compounds described in U.S. Patent Nos. 8,039,627; 8,357,697; and 8,524,733. Tetrabenazine may be administered by a variety of methods including the formulations disclosed in PCT Publications WO 2010/018408, WO 2011/019956, and WO 2014/047167. In one embodiment, the VMAT2 inhibitor used in the methods described herein is (S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or an isotopic variant, pharmaceutically acceptable salt (*e.g.*, ditosylate salt), or polymorph thereof *(see* U.S. Patent No. 8,039,627). In another embodiment, the VMAT2 inhibitor used in the methods described herein is (2R,3R,11bR)-3-isobutyl-9,10-dimethoxy-1,3,4,6,11b-hexahydro-2H-pyrido[2,1-a]isoquinolin-2-ol (R,R,R DHTBZ) or a precursor thereof. In still another embodiment, the VMAT2 inhibitor used in the methods described herein is [(2R,3 S,11bR)-9,10-Dimethoxy-3-(2-methylpropyl)-1H,2H,3H,4H,6H,7H,11bH-pyrido[2,1-a]isoquinolin-2-yl]methanol (also called Compound 5-1 herein, *see* PCT Application No. PCT/US2016/016892), or a precursor thereof *(e.g.,* a prodrug of Compound 5-1, *see,* PCT Application No. PCT/LTS2016/016892). In yet another embodiment, the VMAT2 inhibitor is tetrabenazine or deuterated tetrabenazine. Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ) *(see,* U.S. Patent 8,524,733). The d₆-TBZ may be administered by a variety of methods including the formulations as disclosed in PCT Publication WO 2014/047167. As described herein, any one of the VMAT2 inhibitors may be combined with a pharmaceutically acceptable excipient, carrier, and/or diluent to form a pharmaceutical composition for use in the methods described herein.

In certain embodiments, the methods described herein for treating treatment-refractory OCD comprise administering TBZ analogs and metabolites, lobeline and analogs, and compounds described in U.S. Patent Nos. 8,039,627; 8,357,697; and 8,524,733. Tetrabenazine may be administered by a variety of methods including the formulations disclosed in PCT Publications WO 2010/018408, WO 2011/019956, and WO 2014/047167. -entirety-. In one embodiment, the VMAT2 inhibitor used in the methods described herein is (S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or an isotopic variant, pharmaceutically acceptable salt (e.g., ditosylate salt), or polymorph thereof *(see* U.S. Patent No. 8,039,627). In another embodiment, the VMAT2 inhibitor used in the methods described herein is (2R,3R,11bR)-3-isobutyl-9,10-dimethoxy-1,3,4,6,7,11b-hexahydro-2H-pyrido[2,1-a]isoquinolin-2-ol (R,R,R DHTBZ) or a precursor thereof. In still another embodiment, the VMAT2 inhibitor used in the methods described herein is [(2R,3S,11bR)-9,10-Dimethoxy-3-(2-methylpropyl)-1H,2H,3H,4H,6H,7H,11bH-pyrido[2,1-a]isoquinolin-2-yl]methanol (also called Compound 5-1 herein, *see* PCT Application No. PCT/US2016/016892), or a precursor thereof *(e.g.,* a prodrug of Compound 5-1, *see* PCT Application No. PCT/US2016/016892). In yet another embodiment, the VMAT2 inhibitor is tetrabenazine or deuterated tetrabenazine. Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ) *(see,* U.S. Patent 8,524,733). The d₆-TBZ may be administered by a variety of methods including the formulations as disclosed in PCT Publication WO 2014/047167. As described herein, any one of the VMAT2 inhibitors may be combined with a pharmaceutically acceptable excipient, carrier, and/or diluent to form a pharmaceutical composition for use in the methods described herein.

In certain embodiments, the methods described herein for treating depression in a mood disorder patient comprise administering TBZ analogs and metabolites, reserpine, lobeline and analogs, and compounds described in U.S. Patent Nos. 8,039,627; 8,357,697; and 8,524,733. Tetrabenazine may be administered by a variety of methods including the formulations disclosed in PCT Publications WO 2010/018408, WO 2011/019956, and WO 2014/047167. In one embodiment, the VMAT2 inhibitor is (S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or an isotopic variant, pharmaceutically acceptable salt (*e.g.*, ditosylate salt), or polymorph thereof *(see* U.S. Patent No. 8,039,627). For example, the VMAT2 inhibitor may be (*S*)-(2*R*,3*R*,11b*R*)-3-isobutyl-9,10-dimethoxy-2,3,4,6,7,11-hexahydro-1H-pyrido[2,1-a]isoquinolin-2-yl 2-amino-3-methylbutanoate di(4-methylbenzenesulfonate), or an isotopic variant thereof, or polymorph thereof. In another embodiment, the VMAT2 inhibitor is (2R,3R,11bR)-3-isobutyl-9,10-dimethoxy-1,3,4,6,7,11b-hexahydro-2H-pyrido[2,1-a]isoquinolin-2-ol (R,R,R DHTBZ), or precursor thereof. In still another embodiment, the VMATs inhibitor is [(2R,3S,11bR)-9,10-Dimethoxy-3-(2-methylpropyl)-1H,2H,3H,4H,6H,7H,11bH-pyrido[2,1-a]isoquinolin-2-yl]methanol (also called Compound 5-1 herein, *see* PCT Application No. PCT/US2016/016892), or a precursor thereof *(e.g.,* a prodrug of Compound 5-1, *see* PCT Application No. PCT/US2016/016892). In yet another embodiment, the VMAT2 inhibitor is tetrabenazine or deuterated tetrabenazine. Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ) *(see,* U.S. Patent 8,524,733). The d₆-TBZ may be administered by a variety of methods including the formulations as disclosed in PCT Publication WO 2014/047167. As described herein, any one of the VMAT2 inhibitors may be combined with a pharmaceutically acceptable excipient, carrier, and/or diluent to form a pharmaceutical composition.

Characterizing the activity of a VMAT2 inhibitor can be readily determined using *in vitro* methods and animal models described in the art and herein *(see, e.g.,* Teng, et al., J. Neurochem. 71, 258-65, 1998; Near, (1986), Near, (1986), Mol. Pharmacol. 30: 252-57).

Persons skilled in the art readily appreciate that such assays and techniques are performed using appropriate negative controls (*e.g.*, vehicle only, diluent only, etc.) and appropriate positive controls. Conditions for a particular in vitro assay include temperature, buffers (including salts, cations, media), and other components, which maintain the integrity of the test agent and reagents used in the assay, and which are familiar to a person skilled in the art and/or which can be readily determined. Determining the effectiveness of a VMAT2 inhibitor in an animal model is typically performed using one or more statistical analyses with which a skilled person will be familiar. By way of example, statistical analyses such as two-way analysis of variance (ANOVA), Fisher's exact test, and/or Bonferroni Test, may be used for determining the statistical significance of differences between animal groups.

Compounds described herein include all polymorphs, prodrugs, isomers (including optical, geometric and tautomeric), salts, solvates and isotopes thereof. With regard to stereoisomers, VMAT2 inhibitors may have chiral centers and may occur as racemates, racemic mixtures and as individual enantiomers or diastereomers. All such isomeric forms are included, including mixtures thereof. Unless specifically indicated, throughout the specification and the appended claims, a given chemical formula or name shall encompass tautomers and all stereo, optical and geometrical isomers (*e.g.*, enantiomers, diastereomers, E/Z isomers, etc.) and racemates thereof as well as mixtures in different proportions of the separate enantiomers, mixtures of diastereomers, or mixtures of any of the foregoing forms when such isomers and enantiomers exist, as well as salts thereof, including pharmaceutically acceptable salts thereof and solvates thereof such as for instance hydrates including solvates of the free compounds or solvates of a salt of the compound.

As used herein, "isotopically enriched" refers to an atom having an isotopic composition other than the natural isotopic composition of that atom. "Isotopically enriched" may also refer to a compound containing at least one atom having an isotopic composition other than the natural isotopic composition of that atom.

With regard to the compounds provided herein, when a particular atomic position is designated as having deuterium or "D," it is understood that the abundance of deuterium at that position is substantially greater than the natural abundance of deuterium, which is about 0.015%. A position designated as having deuterium typically has a minimum isotopic enrichment factor of, in particular embodiments, at least 1000 (15% deuterium incorporation), at least 2000 (30% deuterium incorporation), at least 3000 (45% deuterium incorporation), at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium position.

The isotopic enrichment of the compounds provided herein can be determined using conventional analytical methods known to one of ordinary skill in the art, including mass spectrometry, nuclear magnetic resonance spectroscopy, and crystallography.

Isotopic enrichment (for example, deuteration) of pharmaceuticals to improve pharmacokinetics ("PK"), pharmacodynamics ("PD"), and toxicity profiles, has been demonstrated previously with some classes of drugs. *See*, for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gately et. al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrease the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (*i.e.* the step with the highest transition state energy), substitution of a deuterium for that hydrogen will cause a decrease in the reaction rate and the process will slow down. This phenomenon is known as the Deuterium Kinetic Isotope Effect ("DKIE"). (*See, e.g.*, Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon.

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen, may lead to a similar kinetic isotope effect.

For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens, when sequestered by Phase I enzymes, may bind transiently and re-bind in a variety of conformations prior to the chemical reaction (*e.g.*, oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For many drugs, such oxidations are rapid. These drugs therefore often require the administration of multiple or high daily doses.

Therefore, isotopic enrichment at certain positions of a compound provided herein may produce a detectable KIE that will affect the pharmacokinetic, pharmacologic, and/or toxicological profiles of a compound provided herein in comparison with a similar compound having a natural isotopic composition.

The term "isotopic variant" refers to a therapeutic agent that contains an unnatural proportion of an isotope at one or more of the atoms that constitute such a therapeutic agent. In certain embodiments, an "isotopic variant" of a therapeutic agent contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine 123 (¹²³I), iodine-125 (¹²⁵I), iodine-127 (¹²⁷I), iodine-129 (¹²⁹I), and iodine-131 (¹³¹I). In certain embodiments, an "isotopic variant" of a therapeutic agent contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine 123 (¹²³I), iodine-125 (¹²⁵I), iodine-127 (¹²⁷I), iodine-129 (¹²⁹I), and iodine-131 (¹³¹I).

It will be understood that, in a therapeutic agent, any hydrogen can be ²H, for example, or any carbon can be ¹³C, for example, or any nitrogen can be ¹⁵N, for example, or any oxygen can be ¹⁸O, for example, where feasible according to the judgment of one of skill. In certain embodiments, an "isotopic variant" of a therapeutic agent contains unnatural proportions of deuterium (D).

As used herein, pharmaceutically (or physiologically) acceptable salts refer to derivatives of the described compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. For example, such salts include acetates, ascorbates, benzenesulfonates, benzoates, besylates, bicarbonates, bitartrates, bromides/hydrobromides, Ca-edetates/edetates, camsylates, carbonates, chlorides/hydrochlorides, citrates, edisylates, ethane disulfonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulfonates, mesylates, methylbromides, methylnitrates, methylsulfates, mucates, napsylates, nitrates, oxalates, pamoates, pantothenates, phenylacetates, phosphates/diphosphates, polygalacturonates, propionates, salicylates, stearates subacetates, succinates, sulfamides, sulfates, tannates, tartrates, teoclates, toluenesulfonates, triethiodides, ammonium, benzathines, chloroprocaines, cholines, diethanolamines, ethylenediamines, meglumines and procaines. Further pharmaceutically acceptable salts can be formed with cations from metals like aluminium, calcium, lithium, magnesium, potassium, sodium, zinc, and the like. (*see also, e.g.,* Pharmaceutical Salts, Birge, S.M. et al., J. Pharm. Sci., (1977), 66, 1-19).

In addition, prodrugs are also included with respect to the compounds described herein. Prodrugs are any covalently bonded carriers that release a compound *in vivo* when such prodrug is administered to a patient. Prodrugs are generally prepared by modifying functional groups in a way such that the modification is cleaved, either by routine manipulation or *in vivo*, yielding the parent compound. Prodrugs include, for example, compounds as described herein wherein hydroxy, amine, or acid groups are bonded to any group that, when administered to a subject, cleaves to form the hydroxy, amine or acid groups. Thus, representative examples of prodrugs include (but are not limited to) acetate, formate and benzoate derivatives of alcohol and amine functional groups of a compound. Further, in the case of a carboxylic acid (-COOH), esters may be employed, such as methyl esters, ethyl esters, and the like.

The compounds described herein may exist in a continuum of solid states ranging from fully amorphous to fully crystalline. Furthermore, some of the crystalline forms of the compounds may exist as polymorphs. In addition, some compounds may also form solvates with water or other organic solvents. Where the solvent is water, the solvate is a hydrate. The term solvate is used herein to describe a molecular complex comprising a compound and one or more pharmaceutically acceptable solvent molecules, which present in stoichiometric or non-stoichiometric amount. Suitable solvents include, but are not limited to, water, methanol, ethanol, n-propanol, isopropanol, and acetic acid. In certain embodiments, the solvent is pharmaceutically acceptable. In one embodiment, the complex or aggregate is in a crystalline form. In another embodiment, the complex or aggregate is in a non-crystalline form. Examples of hydrates include, but are not limited to, a hemihydrate, monohydrate, dihydrate, trihydrate, tetrahydrate, and pentahydrate.

The term "crystalline form" of a compound can refer to any crystalline form of the compound as a free acid, the compound as a free base, as an acid addition salt of the compound, an base addition salt of the compound, a complex of the compound, a solvate (including hydrate) of the compound, or a co-crystal of the compound. The term "solid form" of a compound can refer to any crystalline form of the compound or any amorphous form of the compound as a free acid, the compound as a free base, as an acid addition salt of the compound, an base addition salt of the compound, a complex of the compound, or a solvate (including hydrate) of the compound, or a co-precipitation of the compound. In many instances, the terms "crystalline form" and "solid form" can refer to those that are pharmaceutically acceptable, including, for example, those of pharmaceutically acceptable addition salts, pharmaceutically acceptable complexes, pharmaceutically acceptable solvates, pharmaceutically acceptable co-crystals, and pharmaceutically acceptable coprecipitations.

As used herein and unless otherwise indicated, the terms "polymorph" and "polymorphic form" refer to solid crystalline forms of a compound or complex. Different polymorphs of the same compound can exhibit different physical, chemical and/or spectroscopic properties. Different physical properties include, but are not limited to stability (*e.g.*, to heat or light), compressibility and density (important in formulation and product manufacturing), and dissolution rates (which can affect bioavailability). Differences in stability can result from changes in chemical reactivity (*e.g.*, differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (*e.g.*, tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

Polymorphs of a molecule can be obtained by a number of methods known in the art. Such methods include, but are not limited to, melt recrystallization, melt cooling, solvent recrystallization, desolvation, rapid evaporation, rapid cooling, slow cooling, vapor diffusion and sublimation. Polymorphs can be detected, identified, classified and characterized using well-known techniques such as, but not limited to, differential scanning calorimetry (DSC), thermogravimetry (TGA), X-ray powder diffractometry (XRPD), single crystal X-ray diffractometry, vibrational spectroscopy, solution calorimetry, solid state nuclear magnetic resonance (NMR), infrared (IR) spectroscopy, Raman spectroscopy, hot stage optical microscopy, scanning electron microscopy (SEM), electron crystallography and quantitative analysis, particle size analysis (PSA), surface area analysis, solubility, and rate of dissolution.

The compounds described herein in certain embodiments are pharmaceutically acceptable isotopically labeled compounds wherein one or more atoms are replaced by atoms having the same atomic number but a different atomic mass. Examples include ²H (deuterium) and ³H (tritium) for hydrogen, ¹¹C, ¹³C and ¹⁴C for carbon, ³⁶Cl for chlorine, ¹⁸F for fluorine, ¹²³I and ¹²⁵I for iodine, ¹³N and ¹⁵N for nitrogen, and ³⁵S for sulfur. Examples also include the substitution of deuterium for ¹H, wherein the deuterium(s) are selectively added to the molecule to alter the metabolism of the drug resulting in some enhanced property such as an increased half-life.

### Methods of Treatment and Pharmaceutical Preparations and Compositions

Provided herein is a VMAT2 inhibitor for use in methods for treating certain neurological disorders characterized by monoamine imbalance in a subject in need thereof. The term "neurological disorder" or "neurological disease" include but is not limited to mania in mood disorders, depression in mood disorders, Rett syndrome, chorea-acanthocytosis, ASD, agitation in Alzheimer's disease, treatment refractory OCD, Lesch-Nyhan syndrome, FXS, and FXTAS.

In certain embodiments, a mood disorder is bipolar disorder, including Bipolar I Disorder, Bipolar II Disorder, Bipolar Disorder Not Otherwise Specified, and cyclothymia.

The VMAT2 inhibitor may prevent, reduce likelihood of occurrence of, slow progression of, delay manifestation of, or treat a symptom associated with mania in a mood disorder, *e.g.*, bipolar disorder. In certain embodiments, the mania in a mood disorder treated according to the methods described herein includes a hypomania. In other embodiments, the mania in a mood disorder treated according to the methods described herein includes severe mania. In a subject with a mood disorder who is experiencing a manic phase, treatment with a VMAT2 inhibitor may improve or effectively reduce one or more moods or behavioral problems associated with mania in a mood disorder, including, by way of example, overly excited or joyful state, extreme irritability, racing thoughts, flight of ideas, psychomotor agitation, talking very fast, increased talkativeness, increased restlessness, increase in goal-directed activity, decreased need for sleep, inflated self-esteem or grandiosity, impulsive behavior, and excessive involvement in pleasurable activities that have a high potential for painful consequence. VMAT2 inhibition results in modulation of the neurotransmitter systems (*e.g.*, dopamine and serotonin), dysregulation of which is associated with mania, and, as such, a reduction in intensity, frequency and amplitude of moods or behaviors associated with mania in a mood disorder would be measurable on a variety of clinical assessment scales.
Provided herein is a VMAT2 inhibitor for use in methods for treating treatment-refractory OCD in a subject in need. The VMAT2 inhibitor may reduce the frequency or severity of one or more obsessive-compulsive behaviors, including cleaning, hoarding, a counting ritual, a checking ritual, a line-crossing obsession, a prayer ritual, a hand washing ritual, following a strict routine, orderliness, requesting reassurance, or a combination thereof. In certain embodiments, the obsessive-compulsive behavior treated according to the methods described herein includes a line-crossing obsession. In other embodiments, the obsessive-compulsive behavior treated is a prayer ritual. In yet other embodiments, the obsessive-compulsive behavior treated is a hand washing ritual. VMAT2 inhibition results in modulation of the neurotransmitter systems (*e.g.*, dopamine and serotonin), which appear to be connected with the pathophysiology of OCD and, as such, a reduction in frequency and severity of the various OCD behavior intensity, frequency, and amplitude would be measurable on a variety of clinical assessment scales.

Provided herein is a VMAT2 inhibitor for use in methods for treating depression in a mood disorder patient in a subject in need thereof. The VMAT2 inhibitor may prevent (*i.e.,* reduce likelihood of occurrence of), slow progression of, delay, or treat depression. Common symptoms of depression include persistent sadness, anxiety or "empty" mood, sleeping too much or too little, middle of the night or early morning waking, reduced appetite and weight loss or increased appetite and weight gain, loss of pleasure and interest in activities once enjoyed, including sex, restlessness, irritability, persistent physical symptoms that do not respond to treatment (such as chronic pain or digestive disorders), difficulty concentrating, remembering, or making decisions, fatigue or loss of energy, feeling guilty, hopeless or worthless, suicide ideation, or any combination thereof. In certain embodiments, one or more symptoms of agitation is treated by the methods comprising administering a VMAT2 inhibitor. In certain embodiments, the mood disorder treated is bipolar disorder. In other embodiments, the mood disorder is major depressive disorder. A reduction in intensity, frequency and amplitude of moods or behaviors or symptoms associated with depression in a mood disorder patient would be measurable on a variety of clinical assessment scales.

As understood by a person skilled in the medical art, the terms, "treat" and "treatment," refer to medical management of a disease, disorder, or condition of a subject (*i.e.,* patient) *(see, e.g.,* Stedman's Medical Dictionary). The terms "treatment" and "treating" embraces both preventative, *i.e.*, prophylactic, or therapeutic, i.e. curative and/or palliative, treatment. Thus the terms "treatment" and "treating" comprise therapeutic treatment of patients having already developed the condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods described herein may be used, for instance, as therapeutic treatment over a period of time as well as for chronic therapy. In addition the terms "treatment" and "treating" comprise prophylactic treatment, *i.e.,* a treatment of patients at risk to develop a condition mentioned hereinbefore, thus reducing the risk of occurrence or reoccurrence.

The subject in need of the compositions and methods described herein includes a subject who has been diagnosed by a person skilled in the medical art. Bipolar disorder is characterized by extreme mood swings that include emotional highs (mania or hypomania) and lows (depression). Mood swings may occur a few times a year, several times a week, or even be experienced at the same time. Symptoms of mania or a manic episode include, for example, a long period of feeling "high" or an overly happy or outgoing mood, extreme irritability, talking very fast, racing thoughts, jumping from one idea to another, being easily distracted, increasing activities, being overly restless, sleeping little, having an unrealistic belief in one's abilities, impulsive behavior, and engaging in pleasurable, high-risk behaviors. Severe mania causes significant impairment in a subject's life (work, school, family life, social life) and may require hospitalization. Severe episodes of mania or depression may also exhibit psychotic symptoms, such as hallucinations or delusions. Hypomania, or hypomanic episode, is a part of bipolar II disorder. Hypomanic episodes have the same symptoms as severe mania, except the mood usually isn't severe enough to impair the subject's work or social life or to require hospitalization, and does not present psychotic symptoms. Without proper treatment, hypomania may develop into severe mania or depression

A mood disorder is as defined in the Diagnostic and Statistical Manual of Mental Disorders (DSM) (*e.g.*, DSM-IV). In some embodiments, a mood disorder includes major depressive disorder, bipolar disorder, persistent depressive disorder, cyclothymia, and seasonal affective disorder. Bipolar disorder is diagnosed using guidelines from the DSM. To be diagnosed with bipolar disorder, the symptoms must be a major change from a patient's normal mood or behavior. There are four basic types of bipolar disorder:
1. Bipolar I Disorder-defined by manic or mixed episodes that last at least seven days, or by manic symptoms that are so severe that the person needs immediate hospital care. Usually, depressive episodes occur as well, typically lasting at least 2 weeks.
2. Bipolar II Disorder-defined by a pattern of depressive episodes and hypomanic episodes, but no full-blown manic or mixed episodes.
3. Bipolar Disorder Not Otherwise Specified (BP-NOS)-diagnosed when symptoms of the illness exist but do not meet diagnostic criteria for either bipolar I or II. However, the symptoms are clearly out of the person's normal range of behavior.
4. Cyclothymic Disorder, or Cyclothymia-a mild form of bipolar disorder. People with cyclothymia have episodes of hypomania as well as mild depression for at least 2 years. However, the symptoms do not meet the diagnostic requirements for any other type of bipolar disorder.

The DSM also has specific criteria for the diagnosis of manic and hypomanic episodes. A manic episode is a distinct period of abnormally and persistently elevated, expansive or irritable mood that lasts at least one week (or less than a week if hospitalization is necessary). The episode includes persistently increased goal-directed activity or energy. To be considered a manic episode, the mood disturbance must be severe enough to cause noticeable impairment at work, school, social activities, or relationships; require hospitalization; or trigger psychosis.

A hypomanic episode is a distinct period of abnormally and persistently elevated, expansive or irritable mood that lasts at least four consecutive days. A hypomanic episode is a distinct change in mood and functioning from normal levels and sufficient that it is noticed by other people; does not significantly impair work, school, social activities, or relationships; does not require hospitalization, does not exhibit psychosis; and not caused by alcohol use, drug use, medication, or a medical condition.

A severe form of bipolar disorder is called Rapid-cycling Bipolar Disorder. Rapid cycling occurs when a person has four or more episodes of major depression, mania, hypomania, or mixed states, all within a year. Rapid cycling seems to be more common in people who have their first bipolar episode at a younger age. One study found that people with rapid cycling had their first episode about 4 years earlier-during the mid to late teen years-than people without rapid cycling bipolar disorder.

Other features of bipolar disorder may include anxiety, melancholy, catatonia, and seasonal pattern.

When getting a diagnosis, a physician conducts a physical examination, an interview, and lab tests. While, mood disorders, including bipolar disorder, cannot be diagnosed using a blood test or a brain scan at this time, these tests can help rule out other factors that may contribute to mood problems, such as a stroke, brain tumor, or thyroid condition. If the problems are not caused by other illnesses, a physician, such as a psychiatrist, may conduct a mental health evaluation.

A number of psychiatric scales and tests have been created to help assess mania in mood disorders. The Young Mania Rating Scale (YMRS) is used to measure the severity of manic episodes. The YMRS consists of 11 items; seven items are scored on a 5-point scale (0 to 4) and four items scored on a 9-point scale (0 to 8). Increasing number value indicates increasing severity of described abnormality for each item. The Clinician-Administered Rating Scale for Mania (CARS-M) may be used to assess severity of both manic and psychotic symptoms. The Altman Self-Rating Mania Scale (ASRM) can be used to assess the presence and severity manic and hypomanic symptoms.

Symptoms associated with depression include, but are not limited to, persistent sadness, anxiety or "empty" mood, sleeping too much or too little, middle of the night or early morning waking, reduced appetite and weight loss, or increased appetite and weight gain, loss of pleasure and interest in activities once enjoyed, including sex, restlessness, irritability, persistent physical symptoms that do not respond to treatment (such as chronic pain or digestive disorders), difficulty concentrating, remembering or making decisions, fatigue or loss of energy, feeling guilty, hopeless or worthless, thoughts of suicide or death.

Many things can contribute to clinical depression. For some people, a number of factors seem to be involved, while for others a single factor can cause the illness. Oftentimes, people become depressed for no apparent reason. Causes of depression varied. A biological cause of depression includes too little or too much of certain neurotransmitters. Changes in these brain chemicals may cause or contribute to depression. Negative thinking patterns and low self-esteem increase the likelihood of developing clinical depression. Gender may also influence development of depression, as more women experience depression than men. While the reasons for this are still unclear, they may include the hormonal changes women go through during menstruation, pregnancy, childbirth and menopause. Other reasons may include the stress caused by the multiple responsibilities that women have. Depression is more likely to occur along with certain illnesses, such as heart disease, cancer, Parkinson's disease, diabetes, Alzheimer's disease and hormonal disorder. Side effects of some medications can bring about depression. A family history of depression also increases the risk for developing the illness. Some studies also suggest that a combination of genes and environmental factors work together to increase risk for depression. Situational causes, such as difficult life events, including divorce, financial problems or the death of a loved one can contribute to depression (See, Mental Health America (MHA), http://*www.mentalhealthamerica.net*/*conditions*/*depression*).

A number of psychiatric scales and tests have been created to help assess depression in mood disorders. The Hamilton Depression Rating Scale (HAM-D) is a multiple item questionnaire used to rate the severity of depression by probing mood, feelings of guilt, suicide ideation, insomnia, agitation or retardation, anxiety, weight loss, and somatic symptoms. The Montgomery-Åsberg Depression Rating Scale (MADRS) includes questions on the following symptoms: The questionnaire includes questions on the following symptoms: apparent sadness, reported sadness, inner tension, reduced sleep, reduced appetite, concentration difficulties, lassitude, inability to feel, pessimistic thoughts, suicidal thoughts. Other depression scales include the Beck Depression Inventory (BDI), the Zung Self-Rating Depression Scale, the Wechsler Depression Rating Scale, the Raskin Depression Rating Scale, the Inventory of Depressive Symptomatology (IDS), the Quick Inventory of Depressive Symptomatology (QIDS).

"Treatment-refractory OCD", also referred to as "refractory OCD" or "treatment-resistant OCD" is defined by the failure to respond to two trials of selective serotonin reuptake inhibitors (SSRIs). Diagnostic criteria for OCD are set forth in the Diagnostic and Statistical Manual of Mental Disorders published by the American Psychiatric Association. General criteria required for an OCD diagnosis include: obsessions or compulsions or both; knowledge or lack of knowledge that obsessions and compulsions are excessive or unreasonable; obsessions and compulsions are significantly time-consuming and interfere with daily routine and social or work functioning. The obsessions must meet the following criteria: recurrent, persistent and unwelcome thoughts, impulses or images are intrusive and cause distress; the subject tries to neutralize obsessions with another thought or action; and these behaviors or mental acts are meant to prevent or reduce distress, but they are excessive or not realistically related to the problem they're intended to fix. OCD may also have a waxing and waning course.

The Yale-Brown Obsessive Compulsive Scale (Y-BOCS), a 10-item, clinician-administered scale, is the most widely used rating scale for OCD. The Y-BOCS is designed to rate symptom severity, not to establish a diagnosis. The Y-BOCS provides five rating dimensions for obsessions and compulsions: time spent or occupied; interference with functioning or relationships; degree of distress; resistance; and control (*i.e.*, success in resistance). The 10 Y-BOCS items are each scored on a four-point scale from 0 = "no symptoms" to 4 = "extreme symptoms." The sum of the first five items is a severity index for obsessions, and the sum of the last five items an index for compulsions. A translation of total score into an approximate index of overall severity is:

| | |
|---|---|
| 0-7 | subclinical |
| 8-15 | mild |
| 16-23 | moderate |
| 24-31 | severe |
| 32-40 | extreme |

Generally, subjects that experience a 25% decrease in a Y-BOCS score as mild to moderate improvement, and a decrease of 35-50% score as moderate to marked. In controlled treatment trials, a decrease of greater than or equal to 35% is widely accepted as indicating a clinically meaningful response and translates into a global improvement rating of much or very much improved; many studies, however, accept a lower criterion of greater than or equal to a 25% decrease. Alternative rating scales, such as Y-BOCS II, Leyton Obsessional Inventory, Clark-Beck Obsessive Compulsive Inventory, Hamburg Obsessive-Compulsive Inventory, and National Institute of Mental Health Global Obsessive Compulsive Scale, are also available.

Common themes of obsessions are contamination, pathologic doubt, aggression, harm avoidance, unacceptable urges, distasteful or excessive sexual ideas, religious concerns, fears of offending others, a need to know, hoarding, orderliness, and perfection. Compulsions are designed to prevent a future feared event, but are excessive or not realistically related to the problem they're intended to fix. They bring no pleasure to the subject. Common compulsions include washing, cleaning, checking, a need to ask or to confess, arranging, repeating, hoarding, counting, praying, crossing lines, repeating, and mental rituals.

A subject (or patient) to be treated may be a mammal, including a human or non-human primate. The mammal may be a domesticated animal such as a cow, pig, sheep, goat, horse, rabbit, rat, mouse, cat or a dog. The terms "subject" and "patient" are used interchangeably herein in reference, for example, to a mammalian subject, such as a human subject, in one embodiment, a human.

Therapeutic and/or prophylactic benefit includes, for example, an improved clinical outcome, both therapeutic treatment and prophylactic or preventative measures, wherein the object is to prevent or slow or retard (lessen) an undesired physiological change or disorder, or to prevent or slow or retard (lessen) the expansion or severity of such disorder. As discussed herein, beneficial or desired clinical results from treating a subject include, but are not limited to, abatement, lessening, or alleviation of symptoms that result from or are associated the disease, condition, or disorder to be treated; decreased occurrence of symptoms; improved quality of life; longer disease-free status (*i.e.,* decreasing the likelihood or the propensity that a subject will present symptoms on the basis of which a diagnosis of a disease is made); diminishment of extent of disease; stabilized (*i.e.,* not worsening) state of disease; delay or slowing of disease progression; amelioration or palliation of the disease state; and remission (whether partial or total), whether detectable or undetectable; and/or overall survival. "Treatment" can also mean prolonging survival when compared to expected survival if a subject were not receiving treatment. Subjects in need of treatment include those who already have the condition or disorder as well as subjects prone to have or at risk of developing the disease, condition, or disorder (*e.g.*, depression, mania, obsessive compulsive disorder), and those in which the disease, condition, or disorder is to be prevented (*i.e.,* decreasing the likelihood of occurrence of the disease, disorder, or condition).

A "therapeutically effective amount" generally refers to an amount of a treatment, such as a VMAT2 inhibitor, that (i) treats or prevents the particular disease or condition, (ii) attenuates, ameliorates, or eliminates one or more symptoms of the particular disease or condition, or (iii) prevents or delays the onset of one or more symptoms of the particular disease or condition described herein. Optimal doses may generally be determined using experimental models and/or clinical trials. The optimal dose may depend upon the body mass, weight, blood volume, or age of the subject. In general, the dose range of a compound that is a VMAT2 inhibitor applicable per day is usually from 5.0 to 150 mg per day, and in certain embodiments from 5 to 100 mg per day. In certain embodiments, the dose is 40 mg to 80 mg per day. In certain embodiments, the VMAT2 inhibitor is administered at a daily dosage of about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, or about 80 mg. In certain embodiments, the dose range of a VMAT2 inhibitor for a pediatric patient is at a lower range as compared to an adult dose range and can be determined by a person of skill in the art (e.g., pediatric clinical trials). The dose of the VMAT2 inhibitor included in a composition is sufficient to treat mania in a mood disorder.

For example, the dose of the VMAT2 inhibitor in a composition is sufficient to treat a mania in bipolar disorder, *e.g.,* symptoms associated with mania in bipolar disorder, including, a long period of feeling "high" or an overly happy or outgoing mood, extreme irritability, talking very fast, racing thoughts, jumping from one idea to another, being easily distracted, increasing activities, being overly restless, sleeping little, having an unrealistic belief in one's abilities, impulsive behavior, and engaging in pleasurable, high-risk behaviors (*i.e.,* the dose is a therapeutically effective dose for treating, preventing (*i.e.,* reducing likelihood of occurrence of), slow progression of, delay the onset of mania in bipolar disorder.

In another example, the dose of the VMAT2 inhibitor included in a composition is sufficient to treat treatment-refractory OCD *(i.e.,* the dose is a therapeutically effective dose for treating, preventing *(i.e.,* reducing the likelihood of occurrence of) slow progression of, delay the onset of one or more symptoms or conditions associated with OCD).

In another example, the dose of the VMAT2 inhibitor included in a composition is sufficient to treat depression in a mood disorder patient *(i.e.,* the dose is a therapeutically effective dose for treating, preventing (*i.e.,* reducing likelihood of occurrence of), slow progression of, delay the onset of one or more symptoms or conditions associated with depression).

As used herein, amelioration of the symptoms of a particular disorder by administration of a particular pharmaceutical composition refers to any lessening, whether permanent or temporary, lasting or transient, that can be attributed to or associated with administration of the composition.

In the treatment, prevention, or amelioration of one or more symptoms of mania in mood disorder, treatment refractory OCD, depression in mood disorder, Rett an appropriate dosage level generally is 0.001 to 100 mg per kg patient body weight per day (mg/kg per day), 0.01 to 80 mg/kg per day, -about 0.1 to 50 mg/kg per day, about 0.5 to 25 mg/kg per day, or 1 to 20 mg/kg per day, which may be administered in single or multiple doses. Within this range the dosage may be 0.005 to 0.05, 0.05 to 0.5, or 0.5 to 5.0, 1 to 15, 1 to 20, or 1 to 50 mg/kg per day. In certain embodiments, the dosage level is 0.001 to 100 mg/kg per day. In certain embodiments, the dosage level is from 5.0 to 150 mg per day, and in certain embodiments from 5 to 100 mg per day. In other embodiments, the dosage level is from 25 to 100 mg/kg per day. In certain embodiments, the dosage level is 0.01 to 40 mg/kg per day. In certain embodiments, the dosage level is 0.1 to 80 mg/kg per day. In certain embodiments, the dosage level is 0.1 to about 50 mg/kg per day. In certain embodiments, the dosage level is 0.1 to 40 mg/kg per day. In certain embodiments, the dosage level is 0.5 to about 80 mg/kg per day. In certain embodiments, the dosage level is 0.5 to 40 mg/kg per day. In certain embodiments, the dosage level is 0.5 to 25 mg/kg per day. In certain embodiments, the dosage level is 1 to 80 mg/kg per day. In certain embodiments, the dosage level is 1 to about 75 mg/kg per day. In certain embodiments, the dosage level is 1 to 50 mg/kg per day. In certain embodiments, the dosage level is 1 to 40 mg/kg per day. In certain embodiments, the dosage level is 1 to 25 mg/kg per day.

For oral administration, the pharmaceutical compositions can be provided in the form of tablets or capsules containing 1.0 to 1,000 mg of the active ingredient, particularly about 1, about 5, about 10, about 15, about 20, about 25, about 30, about 40, about 45, about 50, about 75, about 80, about 100, about 150, about 200, about 250, about 300, about 400, about 500, about 600, about 750, about 800, about 900, and about 1,000 mg of the active ingredient for the symptomatic adjustment of the dosage to the patient to be treated. In certain embodiments, the pharmaceutical compositions can be provided in the form of tablets or capsules containing about 100 mg of the active ingredient. In certain embodiments, the pharmaceutical compositions can be provided in the form of tablets or capsules containing about 80 mg of the active ingredient. In certain embodiments, the pharmaceutical compositions can be provided in the form of tablets or capsules containing about 75 mg of the active ingredient. In certain embodiments, the pharmaceutical compositions can be provided in the form of tablets or capsules containing about 50 mg of the active ingredient. In certain embodiments, the pharmaceutical compositions can be provided in the form of tablets or capsules containing about 40 mg of the active ingredient. In certain embodiments, the pharmaceutical compositions can be provided in the form of tablets or capsules containing about 25 mg of the active ingredient. In certain embodiments, the pharmaceutical compositions can be provided in the form of tablets or capsules containing about 5 mg to about 150 mg, about 5 mg to about 100 mg, or about 40 mg to about 80 mg of the active ingredient. The compositions may be administered on a regimen of 1 to 4 times per day, including once, twice, three times, and four times per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The VMAT2 inhibitor is administered at a time and frequency appropriate for treating mania in a mood disorder, treatment-refractory OCD, depression in a mood disorder. The VMAT2 inhibitor may be administered 1, 2, or 3 times a day. The dose of a VMAT2 inhibitor may be dose-titrated in a subject. The VMAT2 inhibitor may be provided in unit dosage forms or multiple-dosage forms. Unit-dosage forms, as used herein, refer to physically discrete units suitable for administration to human and animal subjects and packaged individually as is known in the art. Each unit-dose contains a predetermined quantity of the active ingredient(s) sufficient to produce the desired therapeutic effect, in association with the required pharmaceutical carriers or excipients. Examples of unit-dosage forms include ampouls, syringes, and individually packaged tablets and capsules. Unit dosage forms may be administered in fractions or multiples thereof. A multiple-dosage form is a plurality of identical unit-dosage forms packaged in a single container to be administered in segregated unit-dosage form. Examples of multiple-dosage forms include vials, bottles of tablets or capsules, or bottles of pints or gallons.

The minimum dose that is sufficient to provide effective therapy and minimize toxicity is usually preferred. Subjects may generally be monitored for therapeutic effectiveness by clinical evaluation and by using assays suitable for the condition being treated or prevented, which assays will be familiar to those having ordinary skill in the art and are described herein. The level of a compound that is administered to a subject may be monitored by determining the level of the compound in a biological fluid, for example, in the blood, blood fraction (e.g., serum), and/or in the urine, and/or other biological sample from the subject. Any method practiced in the art to detect the compound may be used to measure the level of compound during the course of a therapeutic regimen. A manic phase or depression in a mood disorder may be monitored and assessed by behavioral and physical assessments and analyses as appropriate for the mood disorder with which a person skilled in the art is familiar. OCD symptoms may be monitored and assessed by behavioral and physical assessments and analyses as appropriate for OCD with which a person skilled in the art is familiar.

The dose of a composition comprising a VMAT2 inhibitor described herein for treating mania in a mood disorder, treatment refractory OCD, depression in a mood disorder, may depend upon the subject's condition, that is, stage of the disease, severity of symptoms caused by the disease, general health status, as well as age, gender, and weight, and other factors apparent to a person skilled in the medical art. Similarly, the dose of the VMAT2 inhibitor compound may be determined according to parameters understood by a person skilled in the art and as described herein.

The VMAT2 inhibitor provided herein may be administered alone, or in combination with one or more other compounds provided herein, one or more other active ingredients. In certain embodiments, the VMAT2 inhibitors provided herein may also be combined or used in combination with other agents useful in the treatment, prevention, or amelioration of one or more symptoms of the diseases or conditions for which the VMAT2 inhibitors provided herein are useful, including mania in mood disorders, treatment refractory OCD, depression in mood disorders and other conditions commonly treated with antipsychotic medication.

For combination therapies involving a VMAT2 inhibitor and one or more additional drugs, the VMAT2 inhibitor may be administered concurrently, separately or in the same formulation as the one or more additional drugs, or sequentially.

Also provided herein are methods for treating depression or mania in a mood disorder subject in combination with antipsychotics or other antidepressants (e.g., SSRI). Methods are provided herein for treating depression or mania in a mood disorder by administering to a subject in need thereof a first generation (*i.e.,* typical) or a second generation (*i.e.,* atypical) antipsychotic drug (*e.g.,* a compound) in combination with a VMAT2 inhibitor. Methods are provided herein for treating depression in a mood disorder by administering to a subject in need thereof a tricyclic antidepressant, monoamine oxidase inhibitor, selective serotonin reuptake inhibitor, atypical antidepressant, cognitive behavioural therapy, transcranial magnetic stimulation, or a combination thereof, in combination with a VMAT2 inhibitor.

The dose of the antipsychotic used typically to treat mania in bipolar disorder may be lower than *(i.e.,* reduced, decreased, less than) the heretofore-described dosing range of the drug alone for effectively treating agitation. In certain embodiments, the dose of the antipsychotic drug that is administered when combined with a VMAT2 inhibitor would not effectively treat the neuropsychiatric disorder if administered alone (*i.e.,* if administered in the absence of the VMAT2 inhibitor). In other words, the combination of the VMAT2 inhibitor and the antipsychotic drug act synergistically in the treatment of agitation. When used in combination with a VMAT2 inhibitor, an antipsychotic drug may be used at a dose that if administered alone would have little or no efficacy in treating the neuropsychiatric disorder, that is, the dose of the antipsychotic drug is subtherapeutic. That is, by combining a VMAT2 inhibitor with a subtherapeutic dose of the antipsychotic drug, the efficacy of the antipsychotic drug is enhanced. By way of example, treatment of the neuropsychiatric disorder or symptoms thereof may provide greater relief of agitation and associated anxiety.

Decreasing the dose of an antipsychotic drug has the beneficial effect of reducing the intensity of or preventing (*i.e.,* decreasing the likelihood or risk of occurrence) one or more side effects of the antipsychotic drug. In one embodiment, such as when a typical antipsychotic drug is used for treating agitation, the likelihood of occurrence of movement disorders may be reduced; the severity or intensity of the movement disorder may be decreased or lessened; or the frequency of occurrence of the movement disorder (or symptom thereof) may be reduced (*i.e.,* decreased, lessened). In another embodiment, such as when an atypical drug is used in combination with a VMAT2 inhibitor for treating a neuropsychiatric disorder or symptoms thereof, the likelihood of occurrence or severity of a metabolic disturbance such as weight gain, glucose intolerance, and risk of atherosclerotic cardiovascular disease may be reduced. In other embodiments, side effects that may be reduced by administering to a subject in need thereof an anti-psychotic (either an atypical or typical antipsychotic) combined with a VMAT inhibitor include one or more of sedation, dry mouth, sexual dysfunction, and cardiac arrhythmias.

A typical antipsychotic drug (*i.e.,* first generation antipsychotic drug). includes any one of fluphenazine, haloperidol, loxapine, molindone, perphenazine, pimozide, sulpiride, thioridazine, or trifluoperazine. An atypical antipsychotic drug (*i.e.,* second generation antipsychotic drug) may be any one of aripiprazole, asenapine, clozapine, iloperidone, olanzapine, paliperidone, quetiapine, risperidone, or ziprasidone. The typical antipsychotic haloperidol and the atypical antipsychotics, olanzapine and risperidone have been used for treating patients with Alzheimer's disease with modest benefits observed, however, with increased cognitive decline, cerebrovascular events, parkinsonism, and death (*see, e.g.,* Ballard et al., Nat. Rev. Neurosci. 7:492-500 (2006); Schneider et al., Am. J. Geriatr. Psychiatry 14:191-210 (2006)). Therefore, reduction of the dose of the antipsychotic by administering concurrently a VMAT2 inhibitor could reduce the potential side effects of the antipsychotic as well as treat agitation.

When a VMAT2 inhibitor is administered for treating mania in bipolar disorder in combination with an antipsychotic, each of the antipsychotic and the VMAT2 inhibitor are administered at a time and frequency appropriate for treating agitation. The VMAT2 inhibitor may be administered 1, 2, or 3 times a day. The antipsychotic drug may be administered 1, 2, or 3 times a day independently or together with the VMAT2 inhibitor. In other embodiments, the antipsychotic is administered every week, every two weeks (approximately 2 times per month), every three weeks, every four weeks (approximately once per month), every 6 weeks, or every 8 weeks. In particular embodiments, the dose of the antipsychotic drug used in combination with a VMAT2 inhibitor may be at least about 10% less, at least about 20% less, at least about 25% less, at least about 30% less, at least about 35% less, at least about 40% less, at least about 45% less, at least about 50% less, at least about 55% less, at least about 60% less, at least about 65% less, at least about 70% less, at least about 75% less, at least about 80% less, at least about 85% less, or at least about 90% less than when used alone. In other certain embodiments, the dose of the antipsychotic drug when used in combination with a VMAT2 inhibitor may be between 10-90% less, 10-20% less, 10-25% less, 20-30% less, 25%-30% less, 25%-40% less, 25%-50% less, 25%-60% less, 25%-75% less, 25%-80% less, 30-40% less, 30-60% less, 40-50% less, 40-60% less, 50-60% less, 50-75% less, 60-70% less, 60-75% less, 70%-80% less, or 80-90% less than when the antipsychotic drug is used alone.

### Pharmacokinetic Properties

VMAT2 inhibitor tetrabenazine, which contains two chiral centers and is a racemic mix of two stereoisomers, is rapidly and extensively metabolized *in vivo* to its reduced form, 3-isobutyl-9,10-dimethoxy-1,3,4,6,7,11b-hexahydro-2H-pyrido[2,1-a]isoquinolin-2-ol, also known as dihydrotetrabenazine (DHTBZ). DHTBZ is thought to exist as four individual isomers: (±)α-DHTBZ and (±) beta-DHTBZ. The (2*R*, 3*R*, 11b*R*)-3-isobutyl-9,10-dimethoxy-1,3,4,6,7,11b-hexahydro-2H-pyrido[2,1-a]isoquinolin-2-ol (R,R,R, DHTBZ) isomer, also known as (+)α-3-isobutyl-9,10-dimethoxy-1,3,4,6,7,11b-hexahydro-2H-pyrido[2,1-a]isoquinolin-2-ol, is believed to be the absolute configuration of the most active metabolite (*see, e.g.,* Kilboum Chirality 1997 9:59-62).

In one aspect, a method for treating mania in a mood disorder (e.g., mania in bipolar disorder), treatment-refractory OCD, or depression in a mood disorder in a subject is provided herein that comprises administering to a subject in need thereof a pharmaceutical composition comprising a VMAT2 inhibitor described herein in an amount sufficient to achieve a maximal blood plasma concentration (Cₘₐₓ) of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of between about 15 ng to about 60 ng per mL plasma and a minimal blood plasma concentration (Cₘᵢₙ) of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of at least 15 ng per mL plasma over an 8 hour period.

Reference to plasma concentration of (2R,3R,11bR)-3-isobutyl-9,10-dimethoxy-1,3,4,6,7,11b-hexahydro-2H-pyrido[2,1-a]isoquinolin-2-ol (R,R,R DHTBZ) in the methods described herein includes both deuterated (2R,3R,11bR)-3-isobutyl-9,10-dimethoxy-1,3,4,6,7,11b-hexahydro-2H-pyrido[2,1-a]isoquinolin-2-ol (R,R,R DHTBZ) and non-deuterated (2R,3R, 11bR)-3-isobutyl-9,10-dimethoxy-1,3,4,6,7,11b-hexahydro-2H-pyrido[2,1-a]isoquinolin-2-ol (R,R,R DHTBZ). It is apparent to a person of skill in the art that if a deuterated VMAT2 inhibitor as described herein is administered to a subject (*e.g*., deuterated tetrabenzine, deuterated (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or deuterated (2R,3R,11bR)-3-isobutyl-9,10-dimethoxy-1,3,4,6,7,11b-hexahydro-2H-pyrido[2,1-a]isoquinolin-2-ol, then deuterated (2R,3R,11bR)-3-isobutyl-9,10-dimethoxy-1,3,4,6,7,11b-hexahydro-2H-pyrido[2,1-a]isoquinolin-2-ol will appear in the subject's blood plasma and is to be measured. If a non-deuterated VMAT2 inhibitor as described herein is administered to a subject (*e.g*., tetrabenzine, (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or (2R,3R,11bR)-3-isobutyl-9,10-dimethoxy-1,3,4,6,7,11b-hexahydro-2H-pyrido[2,1-a]isoquinolin-2-ol, then non-deuterated (2R,3R,11bR)-3-isobutyl-9,10-dimethoxy-1,3,4,6,7,1 lb-hexahydro-2H-pyrido[2,1-a]isoquinolin-2-ol will appear in the subject's blood plasma and is to be measured. If a combination of deuterated and non-deuterated VMAT2 inhibitors as described herein is administered to a subject, then both deuterated and non-deuterated (2R,3R,1 lbR)-3-isobutyl-9,10-dimethoxy-l,3,4,6,7,11b-hexahydro-2H-pyrido[2,1-a]isoquinolin-2-ol will appear in the subject's blood plasma and both are to be measured.

In certain embodiments, the Cₘₐₓ of (2R,3R,11bR)-3-isobutyl-9,10-dimethoxy-1,3,4,6,7,1 lb-hexahydro-2H-pyrido[2,1-a]isoquinolin-2-ol (R,R,R DHTBZ) is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma. In certain embodiments, the Cₘᵢₙ of (2R,3R,11bR)-3-isobutyl-9,10-dimethoxy-1,3,4,6,7, 1 lb-hexahydro-2H-pyrido[2,1-a]isoquinolin-2-ol (R,R,R DHTBZ) is at least 15 ng/mL, at least 20 ng/mL, at least 25 ng/mL, at least 30 ng/mL, or at least 35 ng/mL plasma, over a period of 8 hrs, 12 hrs, 16 hrs, 20 hrs, 24 hrs, 28 hrs, or 32 hrs. In certain embodiments, the Cₘᵢₙ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) is between about 15 ng/mL to about 35 ng/mL.

In one embodiment, the pharmaceutical composition is administered in an amount sufficient to provide a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of approximately at least 33% of the Cₘₐₓ over a 24 hour period. In another embodiment, the pharmaceutical composition is administered in an amount sufficient to provide a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of approximately at least 50% of the Cₘₐₓ over a 24 hour period. In certain particular embodiments, the pharmaceutical composition is administered in an amount sufficient to provide a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of approximately between about at least 33% -50% of the Cₘₐₓ over a 24 hour period.

In other certain embodiments, the pharmaceutical composition is administered in an amount sufficient to provide a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of approximately at least 33% of the Cₘₐₓ over a 12 hour period. In yet another certain embodiment, the pharmaceutical composition is administered in an amount sufficient to provide a Cₘₐₓ of (2R,3R,11bR)-3-isobutyl-9,10-dimethoxy-1,3,4,6,11b-hexahydro-2H-pyrido[2,1-a]isoquinolin-2-ol (R,R,R DHTBZ) of about 15 ng/mL to about 60 ng/mL plasma and a Cₘᵢₙ of approximately at least 50% of the Cₘₐₓ over a 12 hour period. In certain particular embodiments, the pharmaceutical composition is administered in an amount sufficient to provide a Cₘₐₓ of (2R,3R,11bR)-3-isobutyl-9,10-dimethoxy-1,3,4,6,7,11b-hexahydro-2H-pyrido[2,l-a]isoquinolin-2-ol (R,R,R DHTBZ) of about 15 ng/mL to about 60 ng/mL plasma and a Cₘᵢₙ of approximately between about at least 33% -50% of the Cₘₐₓ over a 12 hour period.

In another embodiment, the pharmaceutical composition is administered to a subject in need thereof in an amount that provides a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of between about 5 ng/mL to about 30 ng/mL plasma over a 24 hour period. In yet another embodiment, the pharmaceutical composition is administered to a subject in need thereof in an amount that provides a Cₘₐₓ of (2R,3R,11bR)-3-isobutyl-9,10-dimethoxy-1,3,4,6,7,11b-hexahydro-2H-pyrido[2,1-a]isoquinolin-2-ol (R,R,R DHTBZ) of about 15 ng/mL to about 60 ng/mL plasma and a Cₘᵢₙ of between about 7.5 ng/mL to about 30 ng/mL plasma over a 24 hour period.

In another aspect, a method for treating mania in a mood disorder (*e.g.*, mania in bipolar disorder), treatment-refractory OCD, or depression in a mood disorder, in a subject is provided herein that comprises administering to a subject in need thereof a pharmaceutical composition comprising a VMAT2 inhibitor described herein in an amount sufficient to provide: (i) a therapeutic concentration range of about 15 ng to about 60 ng (2R,3R,1 lbR)-3-isobutyl-9,10-dimethoxy-l,3,4,6,7,11b-hexahydro-2H-pyrido[2,1-a]isoquinolin-2-ol (R,R,R DHTBZ) per mL plasma; and (ii) a threshold concentration of at least 15 ng of (2R,3R,11bR)-3-isobutyl-9,10-dimethoxy-1,3,4,6,7,11b-hexahydro-2H-pyrido[2,1-a]isoquinolin-2-ol (R,R,R DHTBZ) per mL plasma over a period of about 8 hours to about 24 hours.

In certain embodiments, the therapeutic concentration range is 15 ng to 35 ng, to 40 ng, to 45 ng, to 50 ng, or to 55 ng (2R,3R,11bR)-3-isobutyl-9,10-dimethoxy-1,3,4,6,7,11b-hexahydro-2H-pyrido[2,1-a]isoquinolin-2-ol (R,R,R DHTBZ) per mL plasma.

In certain embodiments, the threshold concentration of (2R,3R,11bR)-3-isobutyl-9,10-dimethoxy-1,3,4,6,7,11b-hexahydro-2H-pyrido[2,1-a]isoquinolin-2-ol (R,R,R DHTBZ) is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma, over a period of about 8 hrs, about 12 hrs, about 16 hrs, about 20 hrs, about 24 hrs, about 28 hrs, or about 32 hrs. In a particular embodiment, the threshold concentration of (2R,3R,11bR)-3-isobutyl-9,10-dimethoxy-1,3,4,6,7,11b-hexahydro-2H-pyrido[2,l-a]isoquinolin-2-ol (R,R,R DHTBZ) is between about 15 ng/mL to about 35 ng/mL over a period of about 8 hours to about 24 hours.

Plasma concentrations of (2R,3R,11bR)-3-isobutyl-9,10-dimethoxy-1,3,4,6,7, 1 lb-hexahydro-2H-pyrido[2,l-a]isoquinolin-2-ol (R,R,R DHTBZ), and compounds as disclosed herein may be measured by methods as described in Derangula et al., Biomedical Chromatography 2013 27(6): 792-801, Mehvar et al., Drug Metabolism and Distribution 1987 15(2): 250-55 and generally by tandem mass spectroscopy.

### Pharmaceutical Compositions

The pharmaceutical compositions described herein that comprise at least one of the VMAT2 inhibitor compounds described herein may be administered to a subject in need by any one of several routes that effectively deliver an effective amount of the compound. The pharmaceutical compositions may be administered alone, or in combination with one or more other compounds provided herein, one or more other active ingredients. Such administrative routes include, for example, oral, parenteral, enteral, rectal, intranasal, buccal, sublingual, intramuscular, and transdermal. Compositions administered by these routes of administration and others are described in greater detail herein.

The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see*, Remington: The Science and Practice of Pharmacy, supra; Modified-Release Drug Delivery Technology, Rathbone et al., Eds., Drugs and the Pharmaceutical Science, Marcel Dekker, Inc.: New York, NY, 2002; Vol. 126).

The pharmaceutical composition may further comprise at least one physiologically (or pharmaceutically) acceptable or suitable excipient. Any physiologically or pharmaceutically suitable excipient or carrier (*i.e*., a non-toxic material that does not interfere with the activity of the active ingredient(s)) known to those of ordinary skill in the art for use in pharmaceutical compositions may be employed in the compositions described herein. Exemplary excipients include diluents and carriers that maintain stability and integrity of the compound.

Pharmaceutically acceptable excipients are well known in the pharmaceutical art and described, for example, in Rowe et al., Handbook of Pharmaceutical Excipients: A Comprehensive Guide to Uses, Properties, and Safety, 5th Ed., 2006, and in Remington: The Science and Practice of Pharmacy (Gennaro, 21st Ed. Mack Pub. Co., Easton, PA (2005)). Exemplary pharmaceutically acceptable excipients include sterile saline and phosphate buffered saline at physiological pH. Preservatives, stabilizers, dyes, buffers, and the like may be provided in the pharmaceutical composition. In addition, antioxidants and suspending agents may also be used.

The pharmaceutical compositions may be in the form of a solution. The solution may comprise saline or sterile water, and may optionally include antioxidants, buffers, bacteriostats, and other common additives. Alternatively, they may be in the form of a solid, such as powder, tablets, pills, or the like. A composition comprising any one of the compounds described herein may be formulated for depot injection, sustained or slow release (also called timed release). Such compositions may generally be prepared using well known technology and administered by, for example, oral, rectal or subcutaneous implantation, intramuscular, or by implantation at the desired target site. Sustained-release formulations may contain the compound dispersed in a carrier matrix and/or contained within a reservoir surrounded by a rate controlling membrane. Excipients for use within such formulations are biocompatible, and may also be biodegradable; preferably the formulation provides a relatively constant level of VMAT2 inhibitor compound release. The amount of compound contained within a sustained release formulation depends upon the site of implantation, the rate and expected duration of release, and the nature of the condition to be treated or prevented.

The pharmaceutical compositions provided herein may be provided in solid, semisolid, or liquid dosage forms for oral administration. As used herein, oral administration also include buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pregelatinized starch, and mixtures thereof. The binder or filler may be present from about 50 to about 99% by weight in the pharmaceutical compositions provided herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Vee gum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as com starch, potato starch, tapioca starch, and pre-gelatinized starch; clays; aligns; and mixtures thereof. The amount of disintegrant in the pharmaceutical compositions provided herein varies upon the type of formulation, and is readily discernible to those of ordinary skill in the art. The pharmaceutical compositions provided herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, com oil, and soybean oil; zinc stearate; ethyl oleate; ethyl laureate; agar; starch; lycopodium; silica or silica gels, such as AEROSIL^{®} 200 (W.R. Grace Co., Baltimore, MD) and CAB-0-SIL^{®} (Cabot Co. of Boston, MA); and mixtures thereof. The pharmaceutical compositions provided herein may contain about 0.1 to about 5% by weight of a lubricant.

Suitable glidants include colloidal silicon dioxide, CAB-0-SIL^{®} (Cabot Co. of Boston, MA), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many carriers and excipients may serve several functions, even within the same formulation. The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde (the term "lower" means an alkyl having between 1 and 6 carbon atoms), e.g., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) provided herein, and a dialkylated mono- or polyalkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions provided herein for oral administration may be also provided in the forms of liposomes, micelles, microspheres, or nanosystems. Micellar dosage forms can be prepared as described in U.S. Pat. No. 6,350,458.

The pharmaceutical compositions provided herein may be provided as noneffervescent or effervescent granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms. The pharmaceutical compositions provided herein may be formulated as immediate or modified release dosage forms, including delayed-, sustained, pulsed-, controlled, targeted-, and programmed-release forms.

The pharmaceutical compositions provided herein may be co-formulated with other active ingredients which do not impair the desired therapeutic action, or with substances that supplement the desired action, such as antacids, proton pump inhibitors, and H₂-receptor antagonists.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

### Parenteral Administration

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science *(see,* Remington: The Science and Practice of Pharmacy, supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, com oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1 ,3-butanediol, liquid polyethylene glycol (e.g., polyethylene glycol 300 and polyethylene glycol 400), propylene glycol, glycerin, N-methyl-2-pyrrolidone, dimethylacetamide, and dimethylsulfoxide.

Suitable antimicrobial agents or preservatives include, but are not limited to, phenols, cresols, mercurials, benzyl alcohol, chlorobutanol, methyl and propyl phydroxybenzates, thimerosal, benzalkonium chloride, benzethonium chloride, methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including alpha-cyclodextrin, beta-cyclodextrin, hydroxypropyl-beta-cyclodextrin, sulfobutylether-beta-cyclodextrin, and sulfobutylether 7-beta-cyclodextrin (CAPTISOL^{®}, CyDex, Lenexa, KS).

The pharmaceutical compositions provided herein may be formulated for single or multiple dosage administration. The single dosage formulations are packaged in an ampule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions provided herein may be formulated as immediate or modified release dosage forms, including delayed-, sustained, pulsed-, controlled, targeted-, and programmed-release forms.

The pharmaceutical compositions may be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers, such as hydrogels of esters of acrylic and methacrylic acid, collagen, cross-linked polyvinylalcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### Topical Administration

The pharmaceutical compositions provided herein may be administered topically to the skin, orifices, or mucosa. The topical administration, as used herein, include (intra)dermal, conjuctival, intracorneal, intraocular, ophthalmic, auricular, transdermal, nasal, vaginal, uretheral, respiratory, and rectal administration.

The pharmaceutical compositions provided herein may be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions provided herein may also comprise liposomes, micelles, microspheres, nanosystems, and mixtures thereof.

Pharmaceutically acceptable carriers and excipients suitable for use in the topical formulations provided herein include, but are not limited to, aqueous vehicles, water miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions may also be administered topically by electroporation, iontophoresis, phonophoresis, sonophoresis and microneedle or needle-free injection, such as POWDERJECT^{™} (Chiron Corp., Emeryville, CA), and BIOJECT^{™} (Bioj ect Medical Technologies Inc., Tualatin, OR).

The pharmaceutical compositions provided herein may be provided in the forms of ointments, creams, and gels. Suitable ointment vehicles include oleaginous or hydrocarbon bases, including such as lard, benzoinated lard, olive oil, cottonseed oil, and other oils, white petrolatum; emulsifiable or absorption bases, such as hydrophilic petrolatum, hydroxystearin sulfate, and anhydrous lanolin; water-removable bases, such as hydrophilic ointment; water-soluble ointment bases, including polyethylene glycols of varying molecular weight; emulsion bases, either water-in-oil (W/O) emulsions or oil-in-water (O/W) emulsions, including cetyl alcohol, glyceryl monostearate, lanolin, and stearic acid *(see,* Remington: The Science and Practice of Pharmacy, supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, Carbopol^{®}; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions provided herein may be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: The Science and Practice of Pharmacy, supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable carriers utilized in rectal and vaginal suppositories include vehicles, such as stiffening agents, which produce a melting point in the proximity of body temperature, when formulated with the pharmaceutical compositions provided herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to 3 g.

The pharmaceutical compositions provided herein may be administered ophthalmically in the forms of solutions, suspensions, ointments, emulsions, gel-forming solutions, powders for solutions, gels, ocular inserts, and implants.

The pharmaceutical compositions provided herein may be administered intranasally or by inhalation to the respiratory tract. The pharmaceutical compositions may be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be provided as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient provided herein, a propellant as solvent; and/or a surfactant, such as sorbitan trioleate, oleic acid, or an oligolactic acid.

The pharmaceutical compositions provided herein may be micronized to a size suitable for delivery by inhalation, such as 50 micrometers or less, or 10 micrometers or less. Particles of such sizes may be prepared using a comminuting method known to those skilled in the art, such as spiral jet milling, fluid bed jet milling, supercritical fluid processing to form nanoparticles, high pressure homogenization, or spray drying.

Capsules, blisters and cartridges for use in an inhaler or insufflator may be formulated to contain a powder mix of the pharmaceutical compositions provided herein; a suitable powder base, such as lactose or starch; and a performance modifier, such as *l*-leucine, mannitol, or magnesium stearate. The lactose may be anhydrous or in the form of the monohydrate. Other suitable excipients include dextran, glucose, maltose, sorbitol, xylitol, fructose, sucrose, and trehalose. The pharmaceutical compositions provided herein for inhaled/intranasal administration may further comprise a suitable flavor, such as menthol and levomenthol, or sweeteners, such as saccharin or saccharin sodium.

The pharmaceutical compositions provided herein for topical administration may be formulated to be immediate release or modified release, including delayed-, sustained-, pulsed-, controlled-, targeted, and programmed release.

### Modified Release

The pharmaceutical compositions provided herein may be formulated as a modified release dosage form. As used herein, the term "modified release" refers to a dosage form in which the rate or place of release of the active ingredient(s) is different from that of an immediate dosage form when administered by the same route. Modified release dosage forms include delayed-, extended-, prolonged-, sustained-, pulsatile- or pulsed-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500.

### Matrix Controlled Release Devices

The pharmaceutical compositions provided herein in a modified release dosage form may be fabricated using a matrix controlled release device known to those skilled in the art *(see,* Takada et al. in "Encyclopedia of Controlled Drug Delivery," Vol. 2, Mathiowitz ed., Wiley, 1999).

In one embodiment, the pharmaceutical compositions provided herein in a modified release dosage form is formulated using an erodible matrix device, which is water swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Rohm America, Inc., Piscataway, NJ); poly(2-hydroxyethyl-methacrylate); polylactides; copolymers of L-glutamic acid and ethyl-L-glutamate; degradable lactic acidglycolic acid copolymers; poly-D-(-)-3-hydroxybutyric acid; and other acrylic acid derivatives, such as homopolymers and copolymers of butylmethacrylate, methylmethacrylate, ethylmethacrylate, ethylacrylate, (2-dimethylaminoethyl)methacrylate, and (trimethylaminoethyl)methacrylate chloride.

In another embodiment, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device included, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinylacetate copolymers, ethylene/propylene copolymers, ethylene/ethyl acrylate copolymers, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethyleneterephthalate, natural rubber, silicone rubbers, polydimethylsiloxanes, silicone carbonate copolymers, and; hydrophilic polymers, such as ethyl cellulose, cellulose acetate, crospovidone, and cross-linked partially hydrolyzed polyvinyl acetate,; and fatty compounds, such as carnauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingredient(s) versus the polymer, and other excipients in the compositions.

The pharmaceutical compositions provided herein in a modified release dosage form may be prepared by methods known to those skilled in the art, including direct compression, dry or wet granulation followed by compression, melt-granulation followed by compression.

### Osmotic Controlled Release Devices

The pharmaceutical compositions provided herein in a modified release dosage form may be fabricated using an osmotic controlled release device, including one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents waterswellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

The other class of osmotic agents is osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol,; organic acids, such as ascorbic acid, benzoic acid, fumaric acid, citric acid, maleic acid, sebacic acid, sorbic acid, adipic acid, edetic acid, glutamic acid, p-tolunesulfonic acid, succinic acid, and tartaric acid; urea; and mixtures thereof.

Osmotic agents of different dissolution rates may be employed to influence how rapidly the active ingredient(s) is initially delivered from the dosage form. For example, amorphous sugars, such as Mannogeme EZ (SPI Pharma, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core may also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate CA ethyl carbonate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxlated ethylene-vinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

Semipermeable membrane may also be a hydrophobic microporous membrane, wherein the pores are substantially filled with a gas and are not wetted by the aqueous medium but are permeable to water, as disclosed in U.S. Pat. No. 5,798,119. Such hydrophobic but water- permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes.

The delivery port(s) on the semipermeable membrane may be formed postcoating by mechanical or laser drilling. Delivery port(s) may also be formed in situ by erosion of a plug of water-soluble material or by rupture of a thinner portion of the membrane over an indentation in the core. In addition, delivery ports may be formed during coating process, as in the case of asymmetric membrane coatings of the type disclosed in U.S. Pat. Nos. 5,612,059 and 5,698,220.

The total amount of the active ingredient(s) released and the release rate can substantially by modulated via the thickness and porosity of the semipermeable membrane, the composition of the core, and the number, size, and position of the delivery ports.

The pharmaceutical compositions in an osmotic controlled-release dosage form may further comprise additional conventional excipients as described herein to promote performance or processing of the formulation.

The osmotic controlled-release dosage forms can be prepared according to conventional methods and techniques known to those skilled in the art *(see,* Remington: The Science and Practice of Pharmacy, supra; Santus and Baker, J. Controlled Release 1995, 35, 1-21; Verma et al., Drug Development and Industrial Pharmacy 2000,26, 695-708; Verma et al., J. Controlled Release 2002, 79, 7-27).

In certain embodiments, the pharmaceutical compositions provided herein are formulated as AMT controlled-release dosage form, which comprises an asymmetric osmotic membrane that coats a core comprising the active ingredient(s) and other pharmaceutically acceptable excipients. *See*, U.S. Pat. No. 5,612,059 and WO 2002/17918. The AMT controlled-release dosage forms can be prepared according to conventional methods and techniques known to those skilled in the art, including direct compression, dry granulation, wet granulation, and a dip-coating method.

In certain embodiment, the pharmaceutical compositions provided herein are formulated as ESC controlled-release dosage form, which comprises an osmotic membrane that coats a core comprising the active ingredient(s), hydroxylethyl cellulose, and other pharmaceutically acceptable excipients.

### Multiparticulate Controlled Release Devices

The pharmaceutical compositions provided herein in a modified release dosage form may be fabricated a multiparticulate controlled release device, which comprises a multiplicity of particles, granules, or pellets, ranging from about 10 µm to about 3 mm, about 50 µm to about 2.5 mm, or from about 100 µm to 1 mm in diameter. Such multiparticulates may be made by the processes know to those skilled in the art, including wet-and dry-granulation, extrusion/spheronization, roller-compaction, melt-congealing, and by spray-coating seed cores. *See*, for example, Multiparticulate Oral Drug Delivery; Marcel Dekker: 1994; and Pharmaceutical Pelletization Technology; Marcel Dekker: 1989.

Other excipients as described herein may be blended with the pharmaceutical compositions to aid in processing and forming the multiparticulates. The resulting particles may themselves constitute the multiparticulate device or may be coated by various filmforming materials, such as enteric polymers, water-swellable, and water-soluble polymers. The multiparticulates can be further processed as a capsule or a tablet.

### Targeted Delivery

The pharmaceutical compositions provided herein may also be formulated to be targeted to a particular tissue, receptor, or other area of the body of the subject to be treated, including liposome-, resealed erythrocyte-, and antibody-based delivery systems. Examples include, but are not limited to, U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

The pharmaceutical compositions provided herein may be formulated for single or multiple dosage administration. The single dosage formulations are packaged in an ampule, a vial, or a syringe. The multiple dosage parenteral formulations must contain an antimicrobial agent at bacteriostatic or fungistatic concentrations. All parenteral formulations must be sterile, as known and practiced in the art.

Also provided are kits that comprise one or more unit doses of the VMAT2 inhibitor. A non-limiting example of such a kit includes a blister pack.

The following examples are provided for purposes of illustration, not limitation.

### EXAMPLES

### EXAMPLE 1

### [(2R,3S,11BR)-9,10-DIMETHOXY-3-(2-METHYLPROPYL)-1H,2H,3H,4H,6H,7H,11BH-PYRIDO[2,1-A]ISOQUINOLIN-2-YL]METHANOL

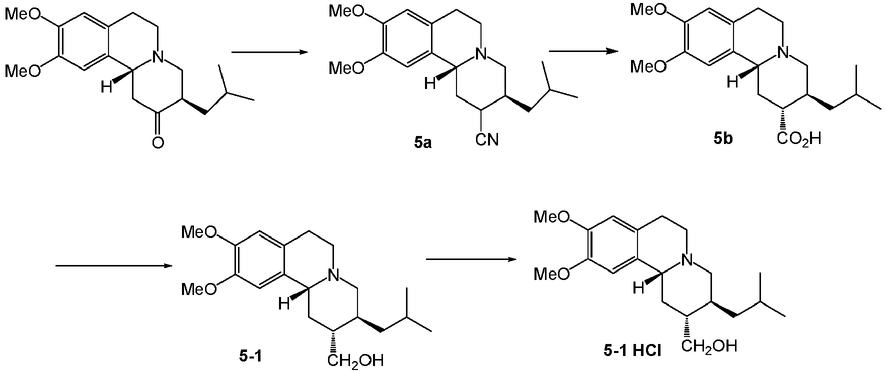

### Step 5A: (3S,11bR)-9,10-Dimethoxy-3-(2-methylpropyl)-1H,2H,3H,4H,6H,7H,11bH-pyrido[2,1-a]isoquinoline-2-carbonitrile

To a 3 L 3-neck round bottomed flask DMSO (1.1 L) and TOSMIC (104 g, 532.5 mmol, 1.3 eq) were charged. To this mixture KO-t-Bu (119.5 g, 1.065 mol) was charged at once at ambient temp (22 °C). An exotherm was observed and the temperature of the mixture increased to 39 °C. Then a suspension of tetrabenazine (130 g, 410 mmol) in DMSO (500 mL) was added to the reaction mixture slowly over 25 min (a slight exotherm observed). EtOH (10.5 mL) was added to this mixture, and the mixture was stirred at ambient temp for 3 h. LC-MS analysis of the mixture revealed presence of ~4:1 ratio of **5a** and starting material. The mixture was poured into cold water (9 L). The mixture was then extracted with EtAOc (4 L). The aqueous layer was extracted with EtOAc (2 L). The combined organics were washed with brine (2 L), dried over Na₂SO₄ and concentrated. The residue was dissolved in acetone (200 ml) and loaded onto a silica column (2 Kg silica gel, packed with hexanes). The column was eluted first with hexanes (2.5 L), followed by 5-20% of acetone in hexanes. The fractions containing 5a and other impurities were combined and concentrated to give an orange oil (72 g), which was dissolved in acetone (100 ml) and loaded onto a silica column (1 Kg silica gel, packed with hexanes). The column was eluted first with hexanes (1 L), followed by 5% of acetone in hexanes (2L), 10% of acetone in hexanes (2L), 15% of acetone in hexanes (2L), and 20% of acetone in hexanes (2L). The fractions containing >90% purity were combined and concentrated to give (3S,11bR)-9,10-dimethoxy-3-(2-methylpropyl)-1H,2H,3H,4H,6H,7H,11bH-pyrido[2,1-a]isoquinoline-2-carbonitrile 5a as an orange solid (61 g, m/z 329.2 [MH⁺]). The fractions containing a mixture of 5a and starting material were collected and concentrated to give 48 g of material, which was dissolved in DMSO (50 ml) and was added to a mixture of TOSMIC (25 g) and KO-t-Bu (28.7 g) in DMSO (250 ml) as shown above. The residue was dissolved in acetone (10 ml) and loaded onto a silica column (600 g silica gel, packed with hexanes). The column was eluted first with hexanes (800 ml), followed by 5-20% of acetone in hexanes. The fractions containing product were combined and concentrated to give orange solid 5a (33 g).

### Step 5B: (3S,11bR)-9,10-Dimethoxy-3-(2-methylpropyl)-1H,2H,3H,4H,6H,7H,11bH-pyrido[2,1-a]isoquinoline-2-carboxylic acid

A 1 gallon pressure reactor was charged with a suspension of 5a (94 g, 286 mmol) in methanol (940 ml) and NaOH (343 g, 8.6 mol) in water (940 ml). This mixture was stirred at 120 °C (internal temp) for 67 h. The mixture was cooled to room temperature and transferred to a round bottom flask. The mixture was concentrated in a rotavap to ~1 L. The mixture was then adjusted pH to 7 using aqueous 6N HCl under cooling. The mixture was extracted with DCM (2 x 3 L and 1 × 2 L). The combined organics were dried over Na₂SO₄ and concentrated to give a dark residue (88 g). The dark residue was taken in acetonitrile (500 ml) and stirred for 30 min. The mixture was filtered and the solid was washed with acetonitrile (50 ml). The solid was dried under vacuum for 2 hours to afford light brown solid (42 g, 49%). This solid was combined with the filtrate and concentrated to a residue. The residue was dissolved in DCM (150 ml) and loaded onto a silica column packed with DCM. The column was eluted with 0-25% of methanol in DCM. The fractions containing product were combined and concentrated to give (3S,11bR)-9,10-Dimethoxy-3-(2-methylpropyl)-1H,2H,3H,4H,6H,7H,11bH-pyrido[2,1-a]isoquinoline-2-carboxylic acid 5b as a pale brown solid (71 g, 71% yield, 92% purity, m/z 348.2 [MH⁺]).

### Step 5C: [(2R,3S,11bR)-9,10-Dimethoxy-3-(2-methylpropyl)-1H,2H,3H,4H,6H,7H,11bH-pyrido[2,1-a]isoquinolin-2-yl]methanol

A 3L round bottom flask was charged with 5b (73.5 g, 211.5 mmol) and THF (1.48 L). This mixture was stirred and cooled to 10 °C (internal temp). To this mixture was added 1 M LAH in THF (423 ml, 423 mmol) slowly over 20 min keeping the temp below 20 °C. The cooling bath was removed, and the mixture was warmed up to room temp. The mixture was heated to 55 °C and stirred for 30 min. The mixture was cooled to room temp and then to 10 °C. EtOAc (30 ml) was added slowly to quench un-reacted LAH followed by ethanol (30 ml). Then water (150 ml) was added to this mixture. The mixture was then concentrated to remove most of organic solvents. Then the mixture was diluted with water (700 ml) and DCM (1 L). The suspension was filtered through a pad of celite. The filtered cake was washed with DCM (2 x 500 ml). The combined filtrates were taken in separatory funnel and the layers separated. The aqueous layer was extracted with DCM (1 L). The combined organics were dried over Na₂SO₄ and concentrated to give a dark residue. The residue was chromatographed on silica column using 0-10% of methanol in DCM as eluent. The fractions containing product were combined and concentrated to afford foamy orange residue. To this residue hexanes (100 ml) was added and concentrated under reduced pressure at 45 °C for 2 h to afford [(2R,3 S,11bR)-9,10-Dimethoxy-3-(2-methylpropyl)-1H,2H,3H,4H,6H,7H,11bH-pyrido[2,1-a]isoquinolin-2-yl]methanol (5-1) (also called Compound 5-1 herein) as a pale brown solid (51 g, 72%, 95% HPLC purity by 220 nm, m/z 334.2 [MH⁺]). This material may be further purified by silica gel chromatography using 0-10% of methanol in DCM or ethyl acetate as eluent.

### Step 5D: [(2R,3S,11bR)-9,10-Dimethoxy-3-(2-methylpropyl)-1H,2H,3H,4H,6H,7H,11bH-pyrido[2,1-a]isoquinolin-2-yl]methanol HCl salt

A 2L round bottom flask was charged with 5-1 (43 g, 129 mmol) and diethyl ether (860 mL). This mixture was stirred and cooled to 15 °C (internal temp). To this mixture was added 2 M HCl in diethyl ether (97 ml, 193 mmol) slowly over 15 min. A white precipitate formed. The cooling bath was removed and the mixture was warmed to room temp. The mixture was then stirred for 45 min. The mixture was filtered and the filtered solid was washed with diethyl ether (100 ml), with MTBE (100 ml) and then with hexanes (100 ml). The solid was then dried in vacuum oven at 40 °C for 18 h. [(2R,3S,11bR)-9,10-Dimethoxy-3-(2-methylpropyl)-1H,2H,3H,4H,6H,7H,11bH-pyrido[2,1-a]isoquinolin-2-yl]methanol HCl salt (5-1 **HCl**) was isolated as an off-white solid (44.7 g, 94% yield, m/z 334.2 [MH⁺]).

### EXAMPLE 2

### METHODS FOR DETERMINING VMAT2 INHIBITORY ACTIVITY OF A COMPOUND

Examples of techniques for determining the capability of a compound to inhibit VMAT2 are provided below.

The procedure is adapted from that described previously (*see, e.g*., Near, (1986), Mol. Pharmacol. 30: 252-57; Teng, et al., J. Neurochem. 71, 258-65, 1998). Homogenates from human platelets or Sprague-Dawley rat forebrain were prepared by homogenization and then washed by centrifugation as described previously (*see*, *e.g*., Hoare et al., (2003) Peptides 24:1881-97). In a total volume of 0.2 mL in low-binding 96-well plates (Corning #3605), twelve concentrations of Compound 5-1 and R,R,R-DHTBZ were competed against 6 nM ³H-dihydrotetrabenazine (American Radiolabeled Chemicals, Kd 2.6 nM) on rat forebrain homogenate (100 µg membrane protein per well) or human platelet homogenate (50 µg membrane protein per well) in VMAT2 binding buffer (Dulbecco's phosphate buffered saline, 1 mM EDTA, pH 7.4). Following incubation at 25 °C for two hours, bound radioligand was collected by rapid filtration onto GF/B glass fiber filters using a Unifilter-96 Harvester (PerkinElmer). Filter plates were pre-treated for 10 minutes with 0.1% polyethylenimine, and following harvesting the filter plates were washed with 800 µl VMAT2 binding buffer. Bound radioligand was quantified by scintillation counting using a Topcount NXT (PerkinElmer). The results of the competition binding studies are presented below in Table 1 and Table 2.

**Table 1. Rat Forebrain VMAT2 Affinity from Competition Binding Studies**

| Compound | pKi (n) | Ki (nM) |
|---|---|---|
| Compound 5-1 | 8.6 ± 0.1 (2) | 2.6 |
| R,R,R-DHTBZ | 8.7 ± 0.2 (6) | 1.9 |

**Table 2. Human Platelet VMAT2 Affinity from Competition Binding Studies**

| Compound | pKi (n) | Ki (nM) |
|---|---|---|
| Compound 5-1 | 8.3 ± 0.1 (2) | 5.2 |
| R,R,R-DHTBZ | 8.6 ± 0.3 (3) | 2.6 |

Another technique that may be routinely performed to determine the capability of a compound to inhibit VMAT2 is provided below. The following procedure is adapted from a previously described method *(see* Teng, et al., J. Neurochem. 71, 258-65, 1998).

Preparation of rat striatal vesicles: Rat striata from three rats are pooled and homogenized in 0.32 M sucrose. The homogenate is then centrifuged at 2,000 x g for 10 min at 4 °C and the resulting supernatant is centrifuged at 10,000 x g for 30 min at 4 °C. The resulting pellet containing the enriched synaptosomal fraction (2 mL) is subjected to osmotic shock by addition of 7 mL of distilled H₂O, and subsequently the suspension is homogenized. The osmolarity is restored by the addition of 0.9 mL of 0.25 M HEPES and 0.9 mL of 1.0 M neutral L-(+)-tartaric acid dipotassium salt buffer (pH 7.5), followed by a 20 min centrifugation (20,000 x g at 4 °C). The supernatant is then centrifuged for 60 min (55,000 x g at 4 °C) and the resulting supernatant is centrifuged for 45 min (100,000 xg at 4 °C). The resulting pellet is resuspended in 25 mM HEPES, 100 mM L-(+)-tartaric acid dipotassium salt, 5 mM MgCl₂, 10 mM NaCl, 0.05 mM EGTA, pH 7.5 to a protein concentration of 1-2 mg/mL and stored at -80 °C for up to 3 weeks without appreciable loss of binding activity. Immediately before use, the final pellet is resuspended in binding buffer (25 mM HEPES, 100 mM L-(+)-tartaric acid dipotassium salt, 5 mM MgCl₂, 10 mM NaCl, 0.05 mM EGTA, 0.1 mM EDTA, 1.7 mM ascorbic acid, pH 7.4).

[³H]-dihydrotetrabenazine (DHTBZ) Binding: Aliquots of the vesicle suspension (0.16 mL, 15 µg of protein/mL) are incubated with competitor compounds (ranging from 10⁻⁶ to 10⁻¹² M) and 2 nM [³H]-dihydrotetrabenazine (HTBZ; specific activity: 20 Ci/mmol, American Radiolabeled Chemicals, Inc.) for 1 h at room temperature in a total volume of 0.5 mL. The reaction is terminated by rapid filtration of the samples onto Whatman GF/F filters using a Brandel cell harvester. Nonspecific binding is determined using 20 µM tetrabenazine (TBZ). Filters are previously soaked for 2 h with ice-cold polyethyleneimine (0.5%). After the filters are washed three times with the ice-cold buffer, they are placed into scintillation vials with 10 mL scintillation cocktail. Bound radioactivity is determined by scintillation spectrometry.

### EXAMPLE 3

### PHASE II HUMAN CLINICAL TRIAL - VALBENAZINE DITOSYLATE (NBI-98854)

Study Design. Clinical data from tardive dyskinesia (TD) subjects administered repeated doses of ditosylate salt of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, also known as NBI-98854, from 12.5 mg to 100 mg per day indicates the drug is generally well tolerated. A prospective, randomized, double-blind, parallel-group, placebo-controlled phase II clinical trial was conducted in moderate or severe tardive dyskinesia patients with underlying schizophrenia, schizoaffective disorder, or mood disorder, including bipolar disorder and major depressive disorder. Subjects must have been psychiatrically stable as determined clinically by the investigator (or designee). Subjects (n=102) were randomized (1:1) to receive ditosylate salt of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester or placebo administered once daily for 6 weeks. All subjects assigned to the treatment group received 25mg of (S)-2-amino-3-methyl-butyric acid (2R,3R,11bR)-3-isobutyl-9,10-dimethoxy-1,3,4,6,7,1 1b-hexahydro-2H-pyrido[2,1-a]isoquinolin-2-yl ester through week 2, then the dose was titrated to 50 mg or maintained. At week 4, the dose was titrated to 75 mg, maintained, or reduced to the previous dose. The primary endpoint was change-from-baseline (CFB) in the Abnormal Involuntary Movement Scale (AIMS) score at week 6 vs. placebo. The AIMS videos were scored by two central raters who were blinded to study visit sequence and treatment group. Safety assessments included Week 6 change from baseline (CFB) in Young Mania Rating Scale (YMRS) and Montgomery-Asberg Depression Rating Scale (MADRS).

A total of 102 subjects were randomized. 76% of subjects receiving (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester reached the maximum dose of 75 mg. The Safety population was 51 (test group) and 49 (placebo) subjects. Antipsychotics, antidepressants, and anxiolytics were the most common concomitant medications, taken by ≥40% of subjects in each group.

Assessment. Manic symptoms were evaluated using the Young Mania Rating Scale (YMRS) in subjects with underlying mood disorder. Results are shown in Table 3. Manic symptoms improved from baseline to Week 6 for the test group vs. placebo.

**Table 3: Results of Phase II mood disorder patients evaluation using YMRS**

| | Placebo | Valbenazine ditosylate |
|---|---|---|
| Week 6 CFB | -0.3 | -1.1 |

| | | |
|---|---|---|
| CFB: Change from Baseline | | |

Depression measured by MADRS improved from baseline to Week 6 as shown by CFB. Results are shown in Table 4.

**Table 4: Results of Phase II mood disorder patients evaluation using MADRS.**

| | Placebo | Valbenazine ditosylate (75 mg) |
|---|---|---|
| Week 6 CFB | -0.2 | -1.5 |

| | | |
|---|---|---|
| CFB: Change from Baseline | | |

### EXAMPLE 4

### PHASE III HUMAN CLINICAL TRIAL - VALBENAZINE DITOSYLATE (NBI-98854)

Study Design. The efficacy, safety, and tolerability of ditosylate salt of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester for tardive dyskinesia were evaluated in a double-blind, parallel-group, placebo-controlled phase 3 clinical trial in subjects with moderate or severe anti-psychotic-induced tardive dyskinesia and underlying schizophrenia, schizoaffective disorder, or mood disorder. The study design included a double-blind, placebo-controlled treatment period for 6 weeks and a double-blind valbenazine ditosylate treatment period for an additional 42 weeks, for a total of 48 weeks of treatment. Study drug was administered in a double-dummy fashion throughout the 48-week treatment period. Medically stable male and female subjects were enrolled with clinical diagnoses of schizophrenia or schizoaffective disorder with neuroleptic-induced TD or mood disorder with neuroleptic-induced TD.

Subjects were randomized 1: 1: 1 to placebo:40 mg valbenazine ditosylate: 80 mg valbenazine ditosylate on Day -1. Of the total number of subjects, approximately half of subjects who had schizophrenia or schizoaffective disorder were enrolled and all subjects were stratified by underlying disease category to assure balanced randomization among the three treatment groups. Subjects must have been psychiatrically stable as determined clinically by the investigator (or designee).

Valbenazine ditosylate was supplied as capsules containing 40 mg valbenazine ditosylate (dose is of the free base). The doses that were used in this study are: 40 mg qd taken as one valbenazine ditosylate 40 mg capsule and one matching placebo, and 80 mg qd taken as two valbenazine ditosylate 40 mg capsules. Subjects swallowed the capsules with at least 4 oz. of water and were allowed to take the study medication with or without food.

Study drug was self-administered at home (in the presence of the subject's caregiver, if applicable) in the morning between 0700-1000 hours beginning on Day 1. The valbenazine ditosylate 80 mg dose was titrated in a blinded fashion (subjects received 40 mg for the first week followed by 80 mg). The study site called subjects weekly to remind them to take their study medication daily.

At any time, if the subject was unable to tolerate their current dose, the investigator was allowed to decrease the subject's dose. The investigator was allowed to reduce the subject's dose only one time during the study. Subjects who had a dose reduction and were unable to tolerate their "new" dose were discontinued from the study. To maintain the study blind, subjects receiving 40 mg or placebo who had a dose reduction continued to receive their current dose and subjects receiving 80 mg were reduced to 40 mg.

At the end of Week 6 (end of the double-blind placebo-controlled treatment period), subjects entered into the double-blind valbenazine ditosylate treatment period for an additional 42 weeks of treatment. Subjects were re-consented to confirm their willingness to continue in the study. During this treatment period, all subjects received valbenazine ditosylate; however, the subject and investigator remained blinded to actual treatment. Subjects who were initially randomized to placebo were re-randomized (1:1) to receive either valbenazine ditosylate 40 mg or 80 mg and subjects initially randomized to valbenazine ditosylate continued with their current dose. Subjects re-randomized to receive valbenazine ditosylate 80 mg received 40 mg for the first week.

At Week 6, subjects who did not want to continue in the study were discontinued and were asked to return for an early termination visit approximately 4 weeks later. The final posttreatment assessments for subjects who entered the double-blind valbenazine ditosylate treatment period were performed at the end of Week 52 (or early termination).

Efficacy, safety, and pharmacokinetics (PK), were assessed at scheduled times throughout the study. The double-blind, placebo-controlled treatment period visits and the double-blind valbenazine ditosylate treatment period end visit had a visit window of ±3 days. The double-blind valbenazine ditosylate treatment period visits and the follow-up visit had a visit window of ±6 days. Subjects returned to the study center at scheduled visits for study assessments and dispensing of study drug. All study assessments were conducted in the afternoon between 1200-1700 hours.

Study Population. Medically stable male and female subjects with clinical diagnoses of schizophrenia or schizoaffective disorder with neuroleptic-induced tardive dyskinesia (TD) or mood disorder with neuroleptic-induced TD were enrolled. Of the total number of subjects, approximately half of subjects who had schizophrenia or schizoaffective disorder were enrolled and all subjects were stratified by underlying disease category to assure balanced randomization among the three treatment groups. The subjects must have been 18 to 85 years of age (inclusive) and have moderate or severe TD as assessed by a blinded, external AIMS reviewer, based on the subject's AIMS video recording conducted at screening. Subjects must have been psychiatrically stable as determined clinically by the investigator (or designee), including a Brief Psychiatric Rating Scale (BPRS) score of <50 at screening and a Positive and Negative Syndrome Scale (PANSS) total score of <70 at Day -1 (baseline).

Duration of Treatment and Study Participation. The expected duration of study participation for each subject was approximately 58 weeks, including up to 6 weeks of screening, 6 weeks of double-blind, placebo-controlled treatment, 42 weeks of double-blind valbenazine ditosylate treatment, and 4 weeks of follow up assessments or early termination.
Efficacy, safety, and pharmacokinetics (PK), were assessed at scheduled times throughout the study. The double-blind, placebo-controlled treatment period visits (end of Weeks 2, 4, and 6).

Assessment. Manic symptoms were evaluated using the Young Mania Rating Scale (YMRS) in subjects with underlying mood disorder. The YMRS total score and change-from-baseline (CFB) values by visit and treatment group screening at Week 6 for mood disorder subjects is presented in Figure 1. NBI-98854 80mg (no dose reduction) includes subjects randomized to 80 mg who did not have a dose reduction prior to Week 6. Baseline measurement is obtained on Day -1 or Screening if Day -1 is missing. The data indicates that subjects who received treatment with 80 mg of NBI-98854 exhibited reduced mania symptoms vs. placebo as shown by CFB at week 6.

Subjects were also assessed for depressive symptoms using the Montgomery-Asberg Depression Rating Scale (MADRS) conducted using the Structured Interview Guide for the MADRS (SIGMA) in subjects who have mood disorder. Results are shown in Table 5.

**Table 5: Results of Phase III mood disorder patients evaluation using MADRS.**

| | Placebo | Valbenazine ditosylate (80 mg) |
|---|---|---|
| Week 6 CFB | 1.2 (1.2) | -1.5 (0.9) |

| | | |
|---|---|---|
| CFB: Change from Baseline | | |

### EXAMPLE 5

### PHASE IB CLINICAL TRIAL - VALBENAZINE DITOSYLATE (NBI-98854)

Study Design. A Phase 1b, open-label, multiple-dose, trial in pediatric subjects with Tourette Syndrome was conducted for valbenazine ditosylate (NBI-98854). The study was conducted in male and female children (6 to 11 years of age) and male and female adolescents (12 to 18 years of age). Age groups were planned to be divided equally into 3 dosing cohorts with approximately 6 subjects each. Fixed doses of NBI-98854 were administered in separate adolescent dosing cohorts and in separate child dosing cohorts. Study drug was administered in each cohort for 14 consecutive days.

Study drug was administered by designated study staff at the site who supervised its administration while the subject was at the clinic (on Days 1 and 2), study drug was administered by the subject, parent, or legal guardian at home on Days 3 to 13, and could be administered at home (based on the subject's preference) or at the clinic on Day 14. Study drug was administered each morning with breakfast at approximately 0800 hours. The subject could drink water or other liquid after study drug administration.

Assessment. The Children's Yale-Brown Obsessive-Compulsive Scale (CY-BOCS) is a semi-structured interview designed to rate the severity of obsessive and compulsive symptoms in children. The investigator or designee administered the CY-BOCS at screening, baseline (Day -1), on Days 7 and 14 of treatment, and at the final study visit (Day 21 or early termination). Total CY-BOCS score (sum of obsession and compulsion subtotal scores) ≥ 16 at screening suggests that a patient meets syndromal criteria for obsessive compulsive disorder. Ten subjects possessed a total CY-BOC score of ≥ 16 at screening. A decrease of at least 25% in Y-BOCS score is considered at least a partial-response to treatment. Of the ten subjects meeting syndromal criteria for obsessive compulsive disorder, four subjects showed improvement of OCD symptoms of at least ~25% from baseline (Day-1) to Day 14 as measured by total CY-BOCS (Table 6).

**Table 6: Results of Phase Ib OCD patients evaluation using CY-BOCS**

| **Patient** | **CY-BOCS Total Score at Day -1** | **CY-BOCS Total Score at Day 14** |
|---|---|---|
| 1 (child) | 23 | 18 |
| 2 (child) | 22 | 12 |
| 3 (child) | 23 | 17 |
| 4 (adolescent) | 20 | 9 |

## Claims

1. A selective VMAT2 inhibitor for use in a method for treating a disorder in a subject, said method comprising administering to the subject the selective VMAT2 inhibitor, wherein the disorder is selected from mania in a mood disorder or refractory obsessive compulsive disorder (OCD).

2. A selective VMAT2 inhibitor for use according to claim 1, wherein the VMAT2 inhibitor selected from:
tetrabenazine (3-isobutyl-9,10-dimethoxy-1,3,4,6,7,11b-hexahydro-2H-pyrido[2,1-a]isoquinolin-2-one);
(2R,3R,11bR)-3-isobutyl-9,10-dimethoxy-1,3,4,6,7,11b-hexahydro-2H-pyrido[2,1-a]isoquinolin-2-ol (R,R,R DHTBZ);
(S)-2-Amino-3-methyl-butyric acid (2R,3R,11bR)-3-isobutyl-9,10-dimethoxy-1,3,4,6,7, 1 1b-hexahydro-2H-pyrido[2,1-a]isoquinolin-2-yl ester;
[(2R,3S,11bR)-9,10-Dimethoxy-3-(2-methylpropyl)-1H,2H,3H,4H,6H,7H,11bH-pyrido[2,1-a]isoquinolin-2-yl]methanol;
3-isobutyl-9,10-d₆-dimethoxy-1,3,4,6,7,11b-hexahydro-2H-pyrido[2,1-a]isoquinolin-2-one (d₆-TBZ); or
a pharmaceutically acceptable salt, or a polymorph thereof.

3. A selective VMAT2 inhibitor for use in a method for treating a disorder in a subject, wherein the disorder is depression in a mood disorder, said method comprising administering to the subject a selective VMAT2 inhibitor, wherein the VMAT2 inhibitor is selected from:
(2R,3R,11bR)-3-isobutyl-9,10-dimethoxy-1,3,4,6,7,11b-hexahydro-2H-pyrido[2,1-a]isoquinolin-2-ol (R,R,R DHTBZ); or
(S)-2-Amino-3-methyl-butyric acid (2R,3R,11bR)-3-isobutyl-9,10-dimethoxy-1,3,4,6,7, 1 1b-hexahydro-2H-pyrido[2,1-a]isoquinolin-2-yl ester; or
a pharmaceutically acceptable salt, or a polymorph thereof.

4. The selective VMAT2 inhibitor for use according to any one of claims 1-3, wherein the VMAT2 inhibitor is (S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester or a pharmaceutically acceptable salt, or a polymorph thereof.

5. The selective VMAT2 inhibitor for use according to claim 4, wherein the VMAT2 inhibitor is (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or polymorph thereof.

6. The selective VMAT2 inhibitor for use according to any one of claims 1-5, wherein the mood disorder is bipolar disorder.

7. The selective VMAT2 inhibitor for use according to claim 6, wherein the mania in the mood disorder is hypomania or severe mania.

8. The selective VMAT2 inhibitor for use according to claim 3, wherein the mood disorder is major depressive disorder.

9. The selective VMAT2 inhibitor for use according to any one of claims 1-8, wherein the VMAT2 inhibitor is administered at a daily dose of 5 mg to 100 mg.

10. The selective VMAT2 inhibitor for use according to any one of claims 1-9, wherein the VMAT2 inhibitor is administered orally, optionally as a tablet or capsule.

11. The selective VMAT2 inhibitor for use in a method according to any one of claims 1, 2, 4, 5, 9 and 10, wherein the disorder is OCD and the frequency or severity of cleaning, hoarding, a counting ritual, a checking ritual, a line-crossing, a prayer ritual, a hand washing ritual, following a strict routine, orderliness, requesting reassurance, or a combination thereof is reduced.

12. A pharmaceutical composition for use in a method of treating a disorder, said composition comprising a pharmaceutically acceptable excipient and a selective VMAT2 inhibitor; wherein the disorder is selected from mania in a mood disorder or refractory OCD.

13. A pharmaceutical composition for use according to claim 12 wherein the VMAT2 inhibitor selected from:
tetrabenazine (3-isobutyl-9,10-dimethoxy-1,3,4,6,7,11b-hexahydro-2H-pyrido[2,1-a]isoquinolin-2-one);
(2R,3R,11bR)-3-isobutyl-9,10-dimethoxy-1,3,4,6,7,11b-hexahydro-2H-pyrido[2,1-a]isoquinolin-2-ol (R,R,R DHTBZ);
(S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester;
[(2R,3S,11bR)-9,10-Dimethoxy-3-(2-methylpropyl)-1H,2H,3H,4H,6H,7H,11bH-pyrido[2,1-a]isoquinolin-2-yl]methanol;
3-isobutyl-9,10-d₆-dimethoxy-1,3,4,6,7,11b-hexahydro-2H-pyrido[2,1-a]isoquinolin-2-one (d₆-TBZ); or
a pharmaceutically acceptable salt, or a polymorph thereof.

14. A pharmaceutical composition for use in a method of treating a disorder, said composition comprising a pharmaceutically acceptable excipient and a selective VMAT2 inhibitor, wherein the VMAT2 inhibitor is selected from:
(2R,3R,11bR)-3-isobutyl-9,10-dimethoxy-1,3,4,6,7,11b-hexahydro-2H-pyrido[2,1-a]isoquinolin-2-ol (R,R,R DHTBZ); or
(S)-2-Amino-3-methyl-butyric acid (2R,3R,11bR)-3-isobutyl-9,10-dimethoxy-1,3,4,6,7, 1 1b-hexahydro-2H-pyrido[2,1-a]isoquinolin-2-yl ester; or
a pharmaceutically acceptable salt, or a polymorph thereof;
wherein the disorder is depression in a mood disorder.

15. The pharmaceutical composition for use according to any one of claims 12 to 14, wherein the VMAT2 inhibitor is (S)-2-Amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester or a pharmaceutically acceptable salt, or a polymorph thereof.

16. The pharmaceutical composition for use according to claim 15, wherein the VMAT2 inhibitor is (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or polymorph thereof.

17. The pharmaceutical composition for use according to any one of claims 12-16, wherein the mood disorder is bipolar disorder.

18. The pharmaceutical composition for use according to claim 17, wherein the mania in the mood disorder is hypomania or severe mania.

19. The pharmaceutical composition for use according to claim 14, wherein the mood disorder is major depressive disorder.

20. The pharmaceutical composition for use according to any one of claims 12-19, wherein the pharmaceutical composition is formulated as a dosage form having about 5 mg to about 100 mg of the VMAT2 inhibitor, where the disorder is mania in a mood disorder.

21. The pharmaceutical composition for use according to claim 14 or claim 19, wherein the pharmaceutical composition is formulated as a dosage form having about 5 mg to about 100 mg of the VMAT2 inhibitor, where the disorder is depression in a mood disorder.

22. The pharmaceutical composition for use according to claim 12 or claim 13 wherein the pharmaceutical composition is formulated as a dosage form having about 10 mg to about 80 mg of the VMAT2 inhibitor, where the disorder is refractory OCD.

23. The pharmaceutical composition for use according to any one of claims 12-21, wherein the pharmaceutical composition is formulated for oral administration, optionally as a solution, tablet or capsule.

24. The pharmaceutical composition for use according to claim 21 or claim 22, wherein the disorder is OCD and the frequency or severity of cleaning, hoarding, a counting ritual, a checking ritual, a line-crossing, a prayer ritual, a hand washing ritual, following a strict routine, orderliness, requesting reassurance, or a combination thereof is reduced.

## Patentansprüche

1. Selektiver VMAT2-Inhibitor zur Verwendung in einem Verfahren zur Behandlung einer Störung in einem Individuum, wobei das Verfahren das Verabreichen des selektiven VMAT2-Inhibitors an das Individuum umfasst, wobei die Störung aus Manie in einer affektiven Störung oder refraktären Zwangsstörung (OCD) ausgewählt ist.

2. Selektiver VMAT2-Inhibitor zur Verwendung nach Anspruch 1, wobei der VMAT2-Inhibitor aus Folgendem ausgewählt ist:
Tetrabenazin-(3-isobutyl-9,10-dimethoxy-1,3,4,6,7,11b-hexahydro-2H-pyrido[2,1-a]-isochinolin-2-on);
(2R,3R,11bR)-3-Isobutyl-9,10-dimethoxy-1,3,4,6,7,11b-hexahydro-2H-pyrido[2,1-a]-isochinolin-2-ol (R,R,R-DHTBZ);
(S)-2-Amino-3-methylbuttersäure-(2R,3R, 11bR)-3-isobutyl-9,10-dimethoxy-1,3,4,6,7,11b-hexahydro-2H-pyrido[2,1-a]isochinolin-2-ylester;
[(2R,3S,11bR)-9,10-Dimethoxy-3-(2-methylpropyl)-1H,2H,3H,4H,6H,7H,11bH-pyrido-[2,1-a]isochinolin-2-yl]methanol;
3-lsobutyl-9,10-d₆-dimethoxy-1,3,4,6,7,11b-hexahydro-2H-pyrido[2,1-a]isochinolin-2-on (d₆-TBZ); oder
einem pharmazeutisch annehmbaren Salz oder einem Polymorph davon.

3. Selektiver VMAT2-Inhibitor zur Verwendung in einem Verfahren zur Behandlung einer Störung in einem Individuum, wobei die Störung Depression in einer affektiven Störung ist, wobei das Verfahren das Verabreichen eines selektiven VMAT2-Inhibitors an das Individuum umfasst, wobei der VMAT2-Inhibitor aus Folgendem ausgewählt ist:
(2R,3R,11bR)-3-Isobutyl-9,10-dimethoxy-1,3,4,6,7,11b-hexahydro-2H-pyrido[2,1-a]-isochinolin-2-ol (R,R,R-DHTBZ); oder
(S)-2-Amino-3-methylbuttersäure-(2R,3R, 11bR)-3-isobutyl-9,10-dimethoxy-1,3,4,6,7,11b-hexahydro-2H-pyrido[2,1-a]isochinolin-2-ylester; oder
einem pharmazeutisch annehmbaren Salz oder einem Polymorph davon.

4. Selektiver VMAT2-Inhibitor zur Verwendung nach einem der Ansprüche 1 bis 3, wobei der VMAT2-Inhibitor (S)-2-Amino-3-methylbuttersäure-(2R,3R,11bR)-3-isobutyl-9,10-di-methoxy-1,3,4,6,7,11b-hexahydro-2H-pyrido[2,1-a]isochinolin-2-ylester oder ein pharmazeutisch annehmbares Salz oder ein Polymorph davon ist.

5. Selektiver VMAT2-Inhibitor zur Verwendung nach Anspruch 4, wobei der VMAT2-Inhibitor (S)-(2R,3R,11bR)-3-Isobutyl-9,10-dimethoxy-2,3,4,6,7,11b-hexahydro-1H-pyrido[2,1-a]isochinolin-2-yl-2-amino-3-methylbutanoatdi-(4-methylbenzolsulfonat) oder ein Polymorph davon ist.

6. Selektiver VMAT2-Inhibitor zur Verwendung nach einem der Ansprüche 1 bis 5, wobei die affektive Störung eine bipolare Störung ist.

7. Selektiver VMAT2-Inhibitor zur Verwendung nach Anspruch 6, wobei die Manie in der affektiven Störung Hypomanie oder schwere Manie ist.

8. Selektiver VMAT2-Inhibitor zur Verwendung nach Anspruch 3, wobei die affektive Störung eine schwere depressive Störung ist.

9. Selektiver VMAT2-Inhibitor zur Verwendung nach einem der Ansprüche 1 bis 8, wobei der VMAT2-Inhibitor in einer täglichen Dosis von 5 mg bis 100 mg verabreicht wird.

10. Selektiver VMAT2-Inhibitor zur Verwendung nach einem der Ansprüche 1 bis 9, wobei der VMAT2-Inhibitor oral, gegebenenfalls als Tablette oder Kapsel, verabreicht wird.

11. Selektiver VMAT2-Inhibitor zur Verwendung in einem Verfahren nach einem der Ansprüche 1, 2, 4, 5, 9 und 10, wobei die Störung OCD ist und die Häufigkeit oder der Schweregrad des Putzens, Sammelns, eines Zählrituals, eines Prüfrituals, eines Überschreitens einer Linie, eines Betrituals, eines Händewaschrituals, des Befolgens einer strikten Routine, von Ordentlichkeit, des Forderns von Bestätigung oder einer Kombination davon reduziert wird.

12. Pharmazeutische Zusammensetzung zur Verwendung in einem Verfahren zur Behandlung einer Störung, wobei die Zusammensetzung einen pharmazeutisch annehmbaren Exzipienten und einen selektiven VMAT2-Inhibitor umfasst; wobei die Störung aus Manie in einer affektiven Störung oder refraktärer OCD ausgewählt ist.

13. Pharmazeutische Zusammensetzung zur Verwendung nach Anspruch 12, wobei der VMAT2-Inhibitor aus Folgendem ausgewählt ist:
Tetrabenazin-(3-isobutyl-9,10-dimethoxy-1,3,4,6,7,11b-hexahydro-2H-pyrido[2,1-a]-isochinolin-2-on);
(2R,3R,11bR)-3-Isobutyl-9,10-dimethoxy-1,3,4,6,7,11b-hexahydro-2H-pyrido[2,1-a]-isochinolin-2-ol (R,R,R-DHTBZ);
(S)-2-Amino-3-methylbuttersäure-(2R,3R, 11bR)-3-isobutyl-9,10-dimethoxy-1,3,4,6,7,11b-hexahydro-2H-pyrido[2,1-a]isochinolin-2-ylester;
[(2R,3S,11bR)-9,10-Dimethoxy-3-(2-methylpropyl)-1H,2H,3H,4H,6H,7H,11bH-pyrido-[2,1-a]isochinolin-2-yl]methanol;
3-lsobutyl-9,10-d₆-dimethoxy-1,3,4,6,7,11b-hexahydro-2H-pyrido[2,1-a]isochinolin-2-on (d₆-TBZ); oder
einem pharmazeutisch annehmbaren Salz oder einem Polymorph davon.

14. Pharmazeutische Zusammensetzung zur Verwendung in einem Verfahren zur Behandlung einer Störung, wobei die Zusammensetzung einen pharmazeutisch annehmbaren Exzipienten und einen selektiven VMAT2-Inhibitor umfasst, wobei der VMAT2-Inhibitor aus Folgendem ausgewählt ist:
(2R,3R,11bR)-3-Isobutyl-9,10-dimethoxy-1,3,4,6,7,11b-hexahydro-2H-pyrido[2,1-a]-isochinolin-2-ol (R,R,R-DHTBZ); oder
(S)-2-Amino-3-methylbuttersäure-(2R,3R, 11bR)-3-isobutyl-9,10-dimethoxy-1,3,4,6,7,11b-hexahydro-2H-pyrido[2,1-a]isochinolin-2-ylester; oder
einem pharmazeutisch annehmbaren Salz oder einem Polymorph davon;
wobei die Störung Depression in einer affektiven Störung ist.

15. Pharmazeutische Zusammensetzung zur Verwendung nach einem der Ansprüche 12 bis 14, wobei der VMAT2-Inhibitor (S)-2-Amino-3-methylbuttersäure-(2R,3R,11bR)-3-isobutyl-9,10-dimethoxy-1,3,4,6,7,11b-hexahydro-2H-pyrido[2,1-a]isochinolin-2-ylester oder ein pharmazeutisch annehmbares Salz oder ein Polymorph davon ist.

16. Pharmazeutische Zusammensetzung zur Verwendung nach Anspruch 15, wobei der VMAT2-Inhibitor (S)-(2R,3R,11bR)-3-Isobutyl-9,10-dimethoxy-2,3,4,6,7,11b-hexahydro-1H-pyrido[2,1-a]isochinolin-2-yl-2-amino-3-methylbutanoatdi-(4-methylbenzolsulfonat) oder ein Polymorph davon ist.

17. Pharmazeutische Zusammensetzung zur Verwendung nach einem der Ansprüche 12 bis 16, wobei die affektive Störung eine bipolare Störung ist.

18. Pharmazeutische Zusammensetzung zur Verwendung nach Anspruch 17, wobei die Manie in der affektiven Störung Hypomanie oder schwere Manie ist.

19. Pharmazeutische Zusammensetzung zur Verwendung nach Anspruch 14, wobei die affektive Störung eine schwere depressive Störung ist.

20. Pharmazeutische Zusammensetzung zur Verwendung nach einem der Ansprüche 12 bis 19, wobei die pharmazeutische Zusammensetzung als Dosierform mit etwa 5 mg bis etwa 100 mg des VMAT2-Inhibitors formuliert ist, wobei die Störung Manie in einer affektiven Störung ist.

21. Pharmazeutische Zusammensetzung zur Verwendung nach Anspruch 14 oder Anspruch 19, wobei die pharmazeutische Zusammensetzung als Dosierform mit etwa 5 mg bis etwa 100 mg des VMAT2-Inhibitors formuliert ist, wobei die Störung Depression in einer affektiven Störung ist.

22. Pharmazeutische Zusammensetzung zur Verwendung nach Anspruch 12 oder Anspruch 13, wobei die pharmazeutische Zusammensetzung als Dosierform mit etwa 10 mg bis etwa 80 mg des VMAT2-Inhibitors formuliert ist, wobei die Störung refraktäre OCD ist.

23. Pharmazeutische Zusammensetzung zur Verwendung nach einem der Ansprüche 12 bis 21, wobei die pharmazeutische Zusammensetzung zur oralen Verabreichung, gegebenenfalls als Lösung, Tablette oder Kapsel, formuliert ist.

24. Pharmazeutische Zusammensetzung zur Verwendung nach Anspruch 21 oder Anspruch 22, wobei die Störung OCD ist und die Häufigkeit oder der Schweregrad des Putzens, Sammelns, eines Zählrituals, eines Prüfrituals, eines Überschreitens einer Linie, eines Betrituals, eines Händewaschrituals, des Befolgens einer strikten Routine, von Ordentlichkeit, des Forderns von Bestätigung oder einer Kombination davon reduziert wird.

## Revendications

1. Inhibiteur de VMAT2 sélectif pour une utilisation dans une méthode de traitement d'un trouble chez un sujet, ladite méthode comprenant l'administration au sujet de l'inhibiteur de VMAT2 sélectif, dans lequel le trouble est choisi parmi une manie dans le cadre d'un trouble de l'humeur et un trouble obsessionnel compulsif (TOC) réfractaire.

2. Inhibiteur de VMAT2 sélectif pour une utilisation selon la revendication 1, lequel inhibiteur de VMAT2 est choisi parmi :
la tétrabénazine (3-isobutyl-9,10-diméthoxy-1,3,4,6,7,11b-hexahydro-2H-pyrido[2,1-a]isoquinolin-2-one) ;
le (2R,3R,11bR)-3-isobutyl-9,10-diméthoxy-1,3,4,6,7,11b-hexahydro-2H-pyrido[2,1-a]isoquinolin-2- ol (R,R,R DHTBZ) ;
l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ;
le [(2R,3S,11bR)-9,10-diméthoxy-3-(2-méthylpropyl)-1H,2H,3H,4H,6H,7H,11bH-pyrido[2,1-a]isoquinolin-2-yl]méthanol ;
la 3-isobutyl-9,10-d₆-diméthoxy-1,3,4,6,7,11b-hexahydro-2H-pyrido[2,1-a]isoquinolin-2-one (d₆-TBZ) ;
ou un sel pharmaceutiquement acceptable ou un polymorphe de celui-ci.

3. Inhibiteur de VMAT2 sélectif pour une utilisation dans une méthode de traitement d'un trouble chez un sujet, dans lequel le trouble est une dépression dans le cadre d'un trouble de l'humeur, ladite méthode comprenant l'administration au sujet de l'inhibiteur de VMAT2 sélectif, dans lequel l'inhibiteur de VMAT2 est choisi parmi :
le (2R,3R,11bR)-3-isobutyl-9,10-diméthoxy-1,3,4,6,7,11b-hexahydro-2H-pyrido[2,1-a]isoquinolin-2- ol (R,R,R DHTBZ) ; et
l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ;
ou un sel pharmaceutiquement acceptable ou un polymorphe de celui-ci.

4. Inhibiteur de VMAT2 sélectif pour une utilisation selon l'une quelconque des revendications 1 à 3, lequel inhibiteur de VMAT2 est l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ou un sel pharmaceutiquement acceptable ou un polymorphe de celui-ci.

5. Inhibiteur de VMAT2 sélectif pour une utilisation selon la revendication 4, lequel inhibiteur de VMAT2 est le di(4-méthylbenzènesulfonate) de 2-amino-3-méthylbutanoate de (S)-(2R,3R,11bR)-3-isobutyl-9,10-diméthoxy-1,3,4,6,7,11b-hexahydro-2H-pyrido[2,1-a]isoquinolin-2-yle, ou un polymorphe de celui-ci.

6. Inhibiteur de VMAT2 sélectif pour une utilisation selon l'une quelconque des revendications 1 à 5, dans lequel le trouble de l'humeur est un trouble bipolaire.

7. Inhibiteur de VMAT2 sélectif pour une utilisation selon la revendication 6, dans lequel la manie dans le cadre du trouble de l'humeur est une hypomanie ou une manie sévère.

8. Inhibiteur de VMAT2 sélectif pour une utilisation selon la revendication 3, dans lequel le trouble de l'humeur est un trouble dépressif majeur.

9. Inhibiteur de VMAT2 sélectif pour une utilisation selon l'une quelconque des revendications 1 à 8, lequel inhibiteur de VMAT2 est administré à une dose journalière de 5 mg à 100 mg.

10. Inhibiteur de VMAT2 sélectif pour une utilisation selon l'une quelconque des revendications 1 à 9, lequel inhibiteur de VMAT2 est administré par voie orale, éventuellement sous la forme d'un comprimé ou d'une capsule.

11. Inhibiteur de VMAT2 sélectif pour une utilisation selon l'une quelconque des revendications 1, 2, 4, 5, 9 et 10, dans lequel le trouble est un TOC et la fréquence ou la gravité des nettoyages, des accumulations, des rituels de comptage, des rituels de vérification, des franchissements de lignes au sol, des rituels de prière, des rituels de lavage des mains, des suivis d'une routine stricte, de l'ordre, des recherches de réassurances, ou d'une de leurs combinaisons, est réduite.

12. Composition pharmaceutique pour une utilisation dans une méthode de traitement d'un trouble, ladite composition comprenant un excipient pharmaceutiquement acceptable et un inhibiteur de VMAT2 sélectif ; dans laquelle le trouble est choisi parmi une manie dans le cadre d'un trouble de l'humeur et un TOC réfractaire.

13. Composition pharmaceutique pour une utilisation selon la revendication 12, dans laquelle l'inhibiteur de VMAT2 est choisi parmi :
la tétrabénazine (3-isobutyl-9,10-diméthoxy-1,3,4,6,7,11b-hexahydro-2H-pyrido[2,1-a]isoquinolin-2-one) ;
le (2R,3R,11bR)-3-isobutyl-9,10-diméthoxy-1,3,4,6,7,11b-hexahydro-2H-pyrido[2,1-a]isoquinolin-2- ol (R,R,R DHTBZ) ;
l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ;
le (2R,3S,11bR)-9,10-diméthoxy-3-(2-méthylpropyl)-1H,2H,3H,4H,6H,7H,11bH-pyrido[2,1-a]isoquinolin-2-yl]méthanol ;
la 3-isobutyl-9,10-d₆-diméthoxy-1,3,4,6,7,11b-hexahydro-2H-pyrido[2,1-a]isoquinolin-2-one (d₆-TBZ) ;
ou un sel pharmaceutiquement acceptable ou un polymorphe de celui-ci.

14. Composition pharmaceutique pour une utilisation dans une méthode de traitement d'un trouble, ladite composition comprenant un excipient pharmaceutiquement acceptable et un inhibiteur de VMAT2 sélectif, dans laquelle l'inhibiteur de VMAT2 est choisi parmi :
le (2R,3R,11bR)-3-isobutyl-9,10-diméthoxy-1,3,4,6,7,11b-hexahydro-2H-pyrido[2,1-a]isoquinolin-2- ol (R,R,R DHTBZ) ; et
l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ;
ou un sel pharmaceutiquement acceptable ou un polymorphe de celui-ci ;
dans laquelle le trouble est une dépression ou un trouble de l'humeur.

15. Composition pharmaceutique pour une utilisation selon l'une quelconque des revendications 12 à 14, dans laquelle l'inhibiteur de VMAT2 est l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique ou un sel pharmaceutiquement acceptable ou un polymorphe de celui-ci.

16. Composition pharmaceutique pour une utilisation selon la revendication 15, dans laquelle l'inhibiteur de VMAT2 est le di(4-méthylbenzènesulfonate) de 2-amino-3-méthylbutanoate de (S)-(2R,3R,11bR)-3-isobutyl-9,10-diméthoxy-1,3,4,6,7,11b-hexahydro-2H-pyrido[2,1-a]isoquinolin-2-yle, ou un polymorphe de celui-ci.

17. Composition pharmaceutique pour une utilisation selon l'une quelconque des revendications 12 à 16, dans laquelle le trouble de l'humeur est un trouble bipolaire.

18. Composition pharmaceutique pour une utilisation selon la revendication 17, dans laquelle la manie dans le cadre du trouble de l'humeur est une hypomanie ou une manie sévère.

19. Composition pharmaceutique pour une utilisation selon la revendication 14, dans laquelle le trouble de l'humeur est un trouble dépressif majeur.

20. Composition pharmaceutique pour une utilisation selon l'une quelconque des revendications 12 à 19, laquelle composition pharmaceutique est formulée en une forme posologique ayant d'environ 5 mg à environ 100 mg de l'inhibiteur de VMAT2, où le trouble est une manie dans le cadre d'un trouble de l'humeur.

21. Composition pharmaceutique pour une utilisation selon la revendication 14 ou la revendication 19, laquelle composition pharmaceutique est formulée en une forme posologique ayant d'environ 5 mg à environ 100 mg de l'inhibiteur de VMAT2, où le trouble est une dépression dans le cadre d'un trouble de l'humeur.

22. Composition pharmaceutique pour une utilisation selon la revendication 12 ou la revendication 13, laquelle composition pharmaceutique est formulée en une forme posologique ayant d'environ 10 mg à environ 80 mg de l'inhibiteur de VMAT2, où le trouble est un TOC réfractaire.

23. Composition pharmaceutique pour une utilisation selon l'une quelconque des revendications 12 à 21, laquelle composition pharmaceutique est formulée pour une administration par voie orale, éventuellement sous la forme d'une solution, d'un comprimé ou d'une capsule.

24. Composition pharmaceutique pour une utilisation selon la revendication 21 ou la revendication 22, dans laquelle le trouble est un TOC et la fréquence ou la gravité des nettoyages, des accumulations, des rituels de comptage, des rituels de vérification, des franchissements de lignes au sol, des rituels de prière, des rituels de lavage des mains, des suivis d'une routine stricte, de l'ordre, des recherches de réassurances, ou d'une de leurs combinaisons, est réduite.
